# EUROPEAN PATENT APPLICATION

(11) **EP 4 585 619 A1**
(43) Date of publication of application: **16.07.2025**
(21) Application number: 23863405.9
(22) Date of filing: 30.08.2023
(51) Int. Cl.: C07K 16/28, A61P 35/00, A61K 39/00

(54) **ANTI-VISTA MONOCLONAL ANTIBODY AND USE THEREOF**

(30) Priority: 05.09.2022 KR 20220112412
(71) Applicant: Cichlid Inc., Seoul 06105 (KR); Seoul National University R & DB Foundation, Seoul 08826 (KR)
(72) Inventor: LEE, Hun Seok, Seoul 06105 (KR); SHIN, Young Kee, Seoul 08826 (KR); PARK, Hee Geon, Seoul 08826 (KR)
(74) Representative: Heide, Anna Katharina
(86) International application number: PCT/KR2023/012908
(87) International publication number: WO 2024/053929

(57) **Abstract**

The present invention relates to an anti-VISTA monoclonal antibody and a use thereof, and more specifically, to an anti-VISTA monoclonal antibody, a functional fragment thereof, and a use thereof for preventing or treating cancer.

## Description

The present application claims priority to Korean Patent Application No. 10-2022-0112412, filed on September 5, 2022, and the entire disclosure of the aforementioned application is incorporated herein by reference.

The present invention relates to anti-VISTA monoclonal antibodies and uses thereof, and more specifically, to anti-VISTA monoclonal antibodies and their functional fragments, as well as uses thereof for preventing or treating cancer.

### Background of the Invention

Anticancer agents are broadly classified into first-generation chemical anticancer agents, second-generation targeted anticancer agents, and third-generation immune anticancer agents. Unlike the disadvantages of first-generation and second-generation anticancer agents, immune anticancer agents, which belong to third-generation, function by binding to the binding site of cancer cells and T cells to block immune evasion signals mediated by co-inhibitory signal receptors. This mechanism allows T cells, unaffected by the immune evasion mechanism of cancer cells, to destroy cancer cells. In other words, immune anticancer agents have a novel mechanism that enhances the suppressed immune function in the body against cancer cells, leading to the destruction of cancer cells. They have fewer side effects, improve the quality of life of cancer patients, and significantly extend survival periods.

In the environment where cancer exists, along with cancer cells, various immune cells (T cells, NK cells, Tregs, myeloid-derived suppressor cells, dendritic cells, M2 macrophages, M1 macrophages, etc.) and normal cells form the tumor microenvironment (TME). Within this environment, cancer creates conditions that suppress the patient's innate or adaptive immunity, preventing the immune system from attacking the cancer cells. Therefore, methods to eliminate cancer in the TME, such as blocking negative immune checkpoints like PD-1, are being studied to induce the death of cancer cells.

PD-1 (programmed death-1) and its ligands PD-L1/PD-L2 represent another axis of immune negative checkpoints. The PD-1 pathway impairs T cell responses and promotes the induction of Foxp3+ Tregs in the periphery, thereby downregulating tumor-specific immune responses. Consequently, blocking the PD-L1/PD-1 pathway in combination with other immunotherapies inhibits tumor progression. Meanwhile, VISTA (V-domain Ig suppressor of T cell activation), which shares homology with PD-L1, is a novel negative checkpoint ligand that suppresses T cell activation and exhibits a distinct expression pattern.

Accordingly, there is a demand for the development of antibodies that specifically block VISTA as a new immuno-oncology agent.

Accordingly, the present inventors, in the course of researching to develop an antibody having specific binding affinity to human VISTA protein, confirmed that the antibody comprising the unique CDR sequences provided in the present invention exhibits excellent binding neutralization ability against PSGL1 and VSIG3, which bind to VISTA, possesses strong ADCC efficacy, and activates suppressed immune functions, thereby showing remarkable effects in its application as an anticancer agent, and thus completed the present invention.

Therefore, an object of the present invention is to provide an antibody or a fragment thereof that binds to human VISTA protein selected from the group consisting of:
i) an antibody comprising a heavy chain variable region (HV) comprising a complementarity-determining region (CDR) H1 comprising the amino acid sequence represented by SEQ ID NO: 1, a complementarity-determining region (CDR) H2 comprising the amino acid sequence represented by SEQ ID NO: 2, a complementarity-determining region (CDR) H3 comprising the amino acid sequence represented by SEQ ID NO: 3, and a light chain variable region (LV) comprising a complementarity-determining region (CDR) L1 comprising the amino acid sequence represented by SEQ ID NO: 4, a complementarity-determining region (CDR) L2 comprising the amino acid sequence represented by SEQ ID NO: 5, and a complementarity-determining region (CDR) L3 comprising the amino acid sequence represented by SEQ ID NO: 6;
ii) an antibody comprising a heavy chain variable region (variable region in heavy chain, VH) comprising a complementarity-determining region (CDR) H1 comprising the amino acid sequence represented by SEQ ID NO: 9, a complementarity-determining region (CDR) H2 comprising the amino acid sequence represented by SEQ ID NO: 10, a complementarity-determining region (CDR) H3 comprising the amino acid sequence represented by SEQ ID NO: 11, and a light chain variable region (variable region in light chain, VL) comprising a complementarity-determining region (CDR) L1 comprising the amino acid sequence represented by SEQ ID NO: 12, a complementarity-determining region (CDR) L2 comprising the amino acid sequence represented by SEQ ID NO: 13, and a complementarity-determining region (CDR) L3 comprising the amino acid sequence represented by SEQ ID NO: 14;
iii) an antibody comprising a heavy chain variable region (VH) comprising a complementarity-determining region (CDR) H1 comprising the amino acid sequence represented by SEQ ID NO: 17, a complementarity-determining region (CDR) H2 comprising the amino acid sequence represented by SEQ ID NO: 18, a complementarity-determining region (CDR) H3 comprising the amino acid sequence represented by SEQ ID NO: 19, and a light chain variable region (VL) comprising a complementarity-determining region (CDR) L1 comprising the amino acid sequence represented by SEQ ID NO: 20, a complementarity-determining region (CDR) L2 comprising the amino acid sequence represented by SEQ ID NO: 21, and a complementarity-determining region (CDR) L3 comprising the amino acid sequence represented by SEQ ID NO: 22;
iv) an antibody comprising a heavy chain variable region (VH) comprising a complementarity-determining region (CDR) H1 comprising the amino acid sequence represented by SEQ ID NO: 25, a complementarity-determining region (CDR) H2 comprising the amino acid sequence represented by SEQ ID NO: 26, a complementarity-determining region (CDR) H3 comprising the amino acid sequence represented by SEQ ID NO: 27, and a light chain variable region (VL) comprising a complementarity-determining region (CDR) L1 comprising the amino acid sequence represented by SEQ ID NO: 28, a complementarity-determining region (CDR) L2 comprising the amino acid sequence represented by SEQ ID NO: 29, and a complementarity-determining region (CDR) L3 comprising the amino acid sequence represented by SEQ ID NO: 30;
v) an antibody comprising a heavy chain variable region (VH) comprising a complementarity-determining region (CDR) H1 comprising the amino acid sequence represented by SEQ ID NO: 33, a complementarity-determining region (CDR) H2 comprising the amino acid sequence represented by SEQ ID NO: 34, a complementarity-determining region (CDR) H3 comprising the amino acid sequence represented by SEQ ID NO: 35, and a light chain variable region (VL) comprising a complementarity-determining region (CDR) L1 comprising the amino acid sequence represented by SEQ ID NO: 36, a complementarity-determining region (CDR) L2 comprising the amino acid sequence represented by SEQ ID NO: 37, and a complementarity-determining region (CDR) L3 comprising the amino acid sequence represented by SEQ ID NO: 38; and
vi) an antibody comprising a heavy chain variable region (VH) comprising a complementarity-determining region (CDR) H1 comprising the amino acid sequence represented by SEQ ID NO: 41, a complementarity-determining region (CDR) H2 comprising the amino acid sequence represented by SEQ ID NO: 42, a complementarity-determining region (CDR) H3 comprising the amino acid sequence represented by SEQ ID NO: 43, and a light chain variable region (VL) comprising a complementarity-determining region (CDR) L1 comprising the amino acid sequence represented by SEQ ID NO: 44, a complementarity-determining region (CDR) L2 comprising the amino acid sequence represented by SEQ ID NO: 45, and a complementarity-determining region (CDR) L3 comprising the amino acid sequence represented by SEQ ID NO: 46.

Another object of the present invention is to provide a polynucleotide encoding the antibody or fragment thereof, a recombinant vector comprising the same, a transformed cell line comprising the same, and a method for producing the antibody or fragment thereof using the same.

Another object of the present invention is to provide a pharmaceutical composition for preventing or treating cancer comprising the antibody or fragment thereof as an active ingredient.

Another object of the present invention is to provide a pharmaceutical composition for preventing or treating cancer consisting of the antibody or fragment thereof.

Another object of the present invention is to provide a pharmaceutical composition for preventing or treating cancer consisting essentially of the antibody or fragment thereof.

Another object of the present invention is to provide the use of the antibody or fragment thereof for manufacturing a pharmaceutical composition for treating cancer.

Another object of the present invention is to provide a method for treating cancer comprising administering an effective amount of a pharmaceutical composition comprising the antibody or fragment thereof as an active ingredient to a subject in need thereof.

To achieve the above objectives, the present invention provides an antibody or a fragment thereof that binds to human VISTA protein selected from the group consisting of:
i) an antibody comprising a heavy chain variable region (V_{H}) comprising a complementarity determining region (CDR) H1 comprising the amino acid sequence represented by SEQ ID NO: 1, a complementarity determining region (CDR) H2 comprising the amino acid sequence represented by SEQ ID NO: 2, a complementarity determining region (CDR) H3 comprising the amino acid sequence represented by SEQ ID NO: 3, and a light chain variable region (V_{L}) comprising a complementarity determining region (CDR) L1 comprising the amino acid sequence represented by SEQ ID NO: 4, a complementarity determining region (CDR) L2 comprising the amino acid sequence represented by SEQ ID NO: 5, and a complementarity determining region (CDR) L3 comprising the amino acid sequence represented by SEQ ID NO: 6;
ii) an antibody comprising a heavy chain variable region (V_{H}) comprising a complementarity determining region (CDR) H1 comprising the amino acid sequence represented by SEQ ID NO: 9, a complementarity determining region (CDR) H2 comprising the amino acid sequence represented by SEQ ID NO: 10, a complementarity determining region (CDR) H3 comprising the amino acid sequence represented by SEQ ID NO: 11, and a light chain variable region (V_{L}) comprising a complementarity determining region (CDR) L1 comprising the amino acid sequence represented by SEQ ID NO: 12, a complementarity determining region (CDR) L2 comprising the amino acid sequence represented by SEQ ID NO: 13, and a complementarity determining region (CDR) L3 comprising the amino acid sequence represented by SEQ ID NO: 14;
iii) an antibody comprising a heavy chain variable region (V_{H}) comprising a complementarity determining region (CDR) H1 comprising the amino acid sequence represented by SEQ ID NO: 17, a complementarity determining region (CDR) H2 comprising the amino acid sequence represented by SEQ ID NO: 18, a complementarity determining region (CDR) H3 comprising the amino acid sequence represented by SEQ ID NO: 19, and a light chain variable region (V_{L}) comprising a complementarity determining region (CDR) L1 comprising the amino acid sequence represented by SEQ ID NO: 20, a complementarity determining region (CDR) L2 comprising the amino acid sequence represented by SEQ ID NO: 21, and a complementarity determining region (CDR) L3 comprising the amino acid sequence represented by SEQ ID NO: 22;
iv) an antibody comprising a heavy chain variable region (V_{H}) comprising a complementarity determining region (CDR) H1 comprising the amino acid sequence represented by SEQ ID NO: 25, a complementarity determining region (CDR) H2 comprising the amino acid sequence represented by SEQ ID NO: 26, a complementarity determining region (CDR) H3 comprising the amino acid sequence represented by SEQ ID NO: 27, and a light chain variable region (V_{L}) comprising a complementarity determining region (CDR) L1 comprising the amino acid sequence represented by SEQ ID NO: 28, a complementarity determining region (CDR) L2 comprising the amino acid sequence represented by SEQ ID NO: 29, and a complementarity determining region (CDR) L3 comprising the amino acid sequence represented by SEQ ID NO: 30;
v) an antibody comprising a heavy chain variable region (V_{H}) comprising a complementarity determining region (CDR) H1 comprising the amino acid sequence represented by SEQ ID NO: 33, a complementarity determining region (CDR) H2 comprising the amino acid sequence represented by SEQ ID NO: 34, a complementarity determining region (CDR) H3 comprising the amino acid sequence represented by SEQ ID NO: 35, and a light chain variable region (V_{L}) comprising a complementarity determining region (CDR) L1 comprising the amino acid sequence represented by SEQ ID NO: 36, a complementarity determining region (CDR) L2 comprising the amino acid sequence represented by SEQ ID NO: 37, and a complementarity determining region (CDR) L3 comprising the amino acid sequence represented by SEQ ID NO: 38; and
vi) an antibody comprising a heavy chain variable region (V_{H}) comprising a complementarity determining region (CDR) H1 comprising the amino acid sequence represented by SEQ ID NO: 41, a complementarity determining region (CDR) H2 comprising the amino acid sequence represented by SEQ ID NO: 42, a complementarity determining region (CDR) H3 comprising the amino acid sequence represented by SEQ ID NO: 43, and a light chain variable region (V_{L}) comprising a complementarity determining region (CDR) L1 comprising the amino acid sequence represented by SEQ ID NO: 44, a complementarity determining region (CDR) L2 comprising the amino acid sequence represented by SEQ ID NO: 45, and a complementarity determining region (CDR) L3 comprising the amino acid sequence represented by SEQ ID NO: 46.

The present invention also provides a polynucleotide encoding the above antibody or fragment thereof, a recombinant vector comprising the same, a transformed cell line comprising the same, and a method for producing the above antibody or fragment thereof using the same.

To achieve another objective of the present invention, the present invention provides a pharmaceutical composition for preventing or treating cancer comprising the above antibody or fragment thereof as an active ingredient.

Additionally, to achieve another objective of the present invention, the present invention provides a pharmaceutical composition for preventing or treating cancer consisting of the above antibody or fragment thereof.

Additionally, to achieve another objective of the present invention, the present invention provides a pharmaceutical composition for preventing or treating cancer consisting essentially of the above antibody or fragment thereof.

Additionally, to achieve another objective of the present invention, the present invention provides the use of the above antibody or fragment thereof for manufacturing a pharmaceutical composition for treating cancer.

Additionally, to achieve another objective of the present invention, the present invention provides a method for treating cancer comprising administering an effective amount of a pharmaceutical composition comprising the above antibody or fragment thereof as an active ingredient to a subject in need thereof.

Hereinafter, the present invention will be described in detail.

VISTA (V-domain Ig suppressor of T cell activation) is a negative checkpoint ligand that suppresses T cell activation and has homology with PD-L1. It is highly expressed in myeloid cells and Foxp3⁺CD4⁺ cells but is not expressed in tumor cells within the tumor microenvironment (TME). Within the hematopoietic compartment, VISTA is constitutively highly expressed in CD11b^{high} myeloid cells and is expressed at lower levels in CD4⁺, CD8⁺, and Foxp3⁺ Treg cells. VISTA expressed on antigen-presenting cells directly suppresses the proliferation and cytokine production of CD4⁺ and CD8⁺ T cells.

Accordingly, VISTA is considered a novel immune negative checkpoint and a potential target for immuno-oncology therapeutics.

Therefore, the present invention provides an antibody or a fragment thereof that binds to human VISTA protein.

In the present invention, the term 'antibody' is also referred to as immunoglobulin (Ig) and is a general term for proteins that selectively act on antigens and are involved in biological immunity. Naturally occurring whole antibodies are generally composed of two pairs of polypeptides, light chains (LC) and heavy chains (HC), each consisting of multiple domains, or are based on a structure of two pairs of these LC/HC. The types of heavy chains constituting mammalian antibodies are classified into five types represented by the Greek letters α, β, γ, δ, and µ, and depending on the type of heavy chain, different types of antibodies such as IgA, IgD, IgE, IgG, and IgM are formed. The types of light chains constituting mammalian antibodies are classified into two types represented by λ and κ.

The heavy and light chains of an antibody are structurally divided into variable regions and constant regions based on the variability of the amino acid sequence. The constant region of the heavy chain consists of three or four heavy chain constant regions, CH1, CH2, and CH3 (IgA, IgD, and IgG antibodies), and CH4 (IgE and IgM antibodies), depending on the type of antibody, while the light chain consists of one constant region, LC. The heavy and light chains are aligned side by side with their respective variable and constant regions and are connected by a single covalent disulfide bond. The light and heavy chains, as well as the variable regions of the light chains, specifically bind to antigens. Since the whole antibody consists of two pairs of heavy and light chains (HC/LC), a single molecule of the whole antibody has bivalent single specificity, binding to two identical antigens through its two variable regions. The variable region of the antibody that binds to the antigen is referred to as the antigen-binding site of the antibody, and the part of the antigen recognized by the antibody is referred to as the epitope.

The variable region of an antibody containing an antigen-binding site is subdivided into a framework region (FR) with low sequence variability and a complementarity determining region (CDR), which is a hypervariable region with high sequence variability. V_{H} and V_{L} each comprise 2 CDRs and 4 FRs arranged in the order of FR1-CDR1-FR2-CDR2-FR3-CDR3-FR4 from the N-terminus to the C-terminus. Among the variable regions of the antibody, the CDR, which has the highest sequence variability, is the region that directly binds to the antigen and is most important for the antigen specificity of the antibody.

The antibody or fragment of the antibody according to the present invention is not limited in type as long as it has the structure of the aforementioned CDR, V_{H} and V_{L}, or light chain and heavy chain, and the antibody may be in the form of IgG, IgA, IgM, IgE, or IgD, and particularly, it is preferably an IgG antibody. The IgG may include its subtypes such as IgG₁, IgG₂, IgG₃, and IgG₄, but is not limited thereto. In addition, it may be a monoclonal antibody derived from a single B cell or a polyclonal antibody derived from multiple B cells, but it is preferably a monoclonal antibody, which is a population of antibodies with substantially identical amino acid sequences of the heavy chain and light chain. Furthermore, the antibody or its fragment of the present invention may be conjugated with enzymes, fluorescent substances, radioactive substances, proteins, and the like, but is not limited thereto.

The antibody of the present invention may be derived from any animal, including mammals, including humans, and birds, and preferably, it may be derived from humans or may be a chimeric antibody comprising a portion of an antibody derived from humans and a portion of an antibody derived from another species.

In addition, in the present invention, a fragment of an antibody refers to a fragment that retains the antigen-specific binding ability of the whole antibody, and specifically, it may be in the form of Fab, F(ab'), F(ab')₂, Fv, scFv, diabody, or dsFv.

Fab (fragment antigen-binding) is an antigen-binding fragment of an antibody, consisting of one variable domain and one constant domain of each of the heavy chain and light chain. F(ab')₂ is a fragment generated by digesting an antibody with pepsin, in which two Fabs are connected by a disulfide bond at the heavy chain hinge. F(ab') is a monomeric antibody fragment in which the heavy chain hinge is added to the Fab separated by reducing the disulfide bond of the F(ab')₂ fragment. Fv (variable fragment) is an antibody fragment consisting only of the variable regions of the heavy chain and light chain. scFv (single chain variable fragment) is a recombinant antibody fragment in which the heavy chain variable region (V_{H}) and the light chain variable region (V_{L}) are connected by a flexible peptide linker. Diabody is a fragment in which the V_{H} and V_{L} of scFv are connected by a very short linker, preventing them from binding to each other, and the V_{H} and V_{L} of another scFv of the same type bind to form a dimer. dsFv is a polypeptide in which one amino acid residue of each of V_{H} and V_{L} is substituted with a cysteine residue, and the cysteine residues are connected via an S-S bond. The amino acid residue to be substituted with a cysteine residue can be selected based on the prediction of the antibody's three-dimensional structure according to known methods.

In one embodiment of the present invention, the antibody or fragment thereof according to the present invention may be an antibody or fragments thereof that bind to human VISTA protein selected from the group consisting of: i) an antibody comprising a heavy chain variable region (V_{H}) comprising a complementarity determining region (CDR) H1 comprising the amino acid sequence represented by SEQ ID NO: 1, a complementarity determining region (CDR) H2 comprising the amino acid sequence represented by SEQ ID NO: 2, a complementarity determining region (CDR) H3 comprising the amino acid sequence represented by SEQ ID NO: 3, and a light chain variable region (V_{L}) comprising a complementarity determining region (CDR) L1 comprising the amino acid sequence represented by SEQ ID NO: 4, a complementarity determining region (CDR) L2 comprising the amino acid sequence represented by SEQ ID NO: 5, and a complementarity determining region (CDR) L3 comprising the amino acid sequence represented by SEQ ID NO: 6; ii) an antibody comprising a heavy chain variable region (V_{H}) comprising a complementarity determining region (CDR) H1 comprising the amino acid sequence represented by SEQ ID NO: 9, a complementarity determining region (CDR) H2 comprising the amino acid sequence represented by SEQ ID NO: 10, a complementarity determining region (CDR) H3 comprising the amino acid sequence represented by SEQ ID NO: 11, and a light chain variable region (V_{L}) comprising a complementarity determining region (CDR) L1 comprising the amino acid sequence represented by SEQ ID NO: 12, a complementarity determining region (CDR) L2 comprising the amino acid sequence represented by SEQ ID NO: 13, and a complementarity determining region (CDR) L3 comprising the amino acid sequence represented by SEQ ID NO: 14; iii) an antibody comprising a heavy chain variable region (V_{H}) comprising a complementarity determining region (CDR) H1 comprising the amino acid sequence represented by SEQ ID NO: 17, a complementarity determining region (CDR) H2 comprising the amino acid sequence represented by SEQ ID NO: 18, a complementarity determining region (CDR) H3 comprising the amino acid sequence represented by SEQ ID NO: 19, and a light chain variable region (V_{L}) comprising a complementarity determining region (CDR) L1 comprising the amino acid sequence represented by SEQ ID NO: 20, a complementarity determining region (CDR) L2 comprising the amino acid sequence represented by SEQ ID NO: 21, and a complementarity determining region (CDR) L3 comprising the amino acid sequence represented by SEQ ID NO: 22; iv) an antibody comprising a heavy chain variable region (V_{H}) comprising a complementarity determining region (CDR) H1 comprising the amino acid sequence represented by SEQ ID NO: 25, a complementarity determining region (CDR) H2 comprising the amino acid sequence represented by SEQ ID NO: 26, a complementarity determining region (CDR) H3 comprising the amino acid sequence represented by SEQ ID NO: 27, and a light chain variable region (V_{L}) comprising a complementarity determining region (CDR) L1 comprising the amino acid sequence represented by SEQ ID NO: 28, a complementarity determining region (CDR) L2 comprising the amino acid sequence represented by SEQ ID NO: 29, and a complementarity determining region (CDR) L3 comprising the amino acid sequence represented by SEQ ID NO: 30; v) an antibody comprising a heavy chain variable region (V_{H}) comprising a complementarity determining region (CDR) H1 comprising the amino acid sequence represented by SEQ ID NO: 33, a complementarity determining region (CDR) H2 comprising the amino acid sequence represented by SEQ ID NO: 34, a complementarity determining region (CDR) H3 comprising the amino acid sequence represented by SEQ ID NO: 35, and a light chain variable region (V_{L}) comprising a complementarity determining region (CDR) L1 comprising the amino acid sequence represented by SEQ ID NO: 36, a complementarity determining region (CDR) L2 comprising the amino acid sequence represented by SEQ ID NO: 37, and a complementarity determining region (CDR) L3 comprising the amino acid sequence represented by SEQ ID NO: 38; and vi) an antibody comprising a heavy chain variable region (V_{H}) comprising a complementarity determining region (CDR) H1 comprising the amino acid sequence represented by SEQ ID NO: 41, a complementarity determining region (CDR) H2 comprising the amino acid sequence represented by SEQ ID NO: 42, a complementarity determining region (CDR) H3 comprising the amino acid sequence represented by SEQ ID NO: 43, and a light chain variable region (V_{L}) comprising a complementarity determining region (CDR) L1 comprising the amino acid sequence represented by SEQ ID NO: 44, a complementarity determining region (CDR) L2 comprising the amino acid sequence represented by SEQ ID NO: 45, and a complementarity determining region (CDR) L3 comprising the amino acid sequence represented by SEQ ID NO: 46.

In another aspect of the present invention, the antibody or fragment thereof according to the present invention is preferably an antibody or fragments thereof that bind to human VISTA protein selected from the group consisting of: i) an antibody comprising a heavy chain variable region comprising the amino acid sequence from the 1st to 117th of SEQ ID NO: 7 and a light chain variable region comprising the amino acid sequence from the 1st to 108th of SEQ ID NO: 8; ii) an antibody comprising a heavy chain variable region comprising the amino acid sequence from the 1st to 117th of SEQ ID NO: 15 and a light chain variable region comprising the amino acid sequence from the 1st to 108th of SEQ ID NO: 16; iii) an antibody comprising a heavy chain variable region comprising the amino acid sequence from the 1st to 118th of SEQ ID NO: 23 and a light chain variable region comprising the amino acid sequence from the 1st to 107th of SEQ ID NO: 24; iv) an antibody comprising a heavy chain variable region comprising the amino acid sequence from the 1st to 118th of SEQ ID NO: 31 and a light chain variable region comprising the amino acid sequence from the 1st to 107th of SEQ ID NO: 32; v) an antibody comprising a heavy chain variable region comprising the amino acid sequence from the 1st to 115th of SEQ ID NO: 39 and a light chain variable region comprising the amino acid sequence from the 1st to 106th of SEQ ID NO: 40; and vi) an antibody comprising a heavy chain variable region comprising the amino acid sequence from the 1st to 119th of SEQ ID NO: 47 and a light chain variable region comprising the amino acid sequence from the 1st to 106th of SEQ ID NO: 48.

In another aspect of the present invention, the antibody or fragment thereof according to the present invention is more preferably an antibody or fragments thereof that bind to human VISTA protein selected from the group consisting of: i) an antibody comprising a heavy chain consisting of SEQ ID NO: 7 and a light chain consisting of SEQ ID NO: 8; ii) an antibody comprising a heavy chain consisting of SEQ ID NO: 15 and a light chain consisting of SEQ ID NO: 16; iii) an antibody comprising a heavy chain consisting of SEQ ID NO: 23 and a light chain consisting of SEQ ID NO: 24; iv) an antibody comprising a heavy chain consisting of SEQ ID NO: 31 and a light chain consisting of SEQ ID NO: 32; v) an antibody comprising a heavy chain consisting of SEQ ID NO: 39 and a light chain consisting of SEQ ID NO: 40; and vi) an antibody comprising a heavy chain consisting of SEQ ID NO: 47 and a light chain consisting of SEQ ID NO: 48.

The present invention also provides a polynucleotide encoding the antibody or fragment thereof, a recombinant vector comprising the same, a transformed cell line comprising the same, and a method for producing the antibody or fragment thereof using the same.

In the present invention, the term 'polynucleotide' may be described as an oligonucleotide or nucleic acid, and includes DNA molecules (e.g., cDNA or genomic DNA), RNA molecules (e.g., mRNA), analogs of the DNA or RNA generated using nucleotide analogs (e.g., peptide nucleic acids and non-naturally occurring nucleotide analogs), and hybrids thereof. The polynucleotide may be single-stranded or double-stranded. The polynucleotide refers to a nucleotide sequence encoding an antibody comprising a heavy chain and a light chain having a configuration of CDRs specific to human VISTA protein, or a configuration of V_{H} and V_{L}.

The polynucleotide encoding the antibody or antigen-binding fragment thereof of the present invention can be obtained by methods well known in the art. For example, based on the DNA sequence or corresponding amino acid sequence coding for a part or the entirety of the heavy chain and light chain of the antibody, it can be synthesized using oligonucleotide synthesis techniques well known in the art, such as polymerase chain reaction (PCR).

The term 'vector' in the present invention is used for the purpose of cloning or expressing the polynucleotide of the present invention for the recombinant production of the antibody or antigen-binding fragment thereof of the present invention, and generally includes one or more of a signal sequence, an origin of replication, one or more marker genes, enhancer elements, promoters, and transcription termination sequences. The vector of the present invention may preferably be an expression vector, and more preferably, a vector comprising the polynucleotide of the present invention operably linked to a regulatory sequence, such as a promoter.

A plasmid, which is a type of vector, refers to a linear or circular double-stranded DNA molecule to which external polynucleotide fragments can be attached. Other forms of vectors include viral vectors (e.g., replication-defective retroviruses, adenoviruses, and adeno-associated viruses), wherein additional DNA fragments can be introduced into the viral genome. Certain vectors are capable of autonomous replication within the host cells into which they are introduced (e.g., bacterial vectors including bacterial origin and episomal mammalian vectors). Other vectors (e.g., non-episomal mammalian vectors) are integrated into the genome of the host cell upon introduction and are replicated along with the host genome.

In the present invention, the term 'vector' can be understood to have the same meaning as 'expression vector', which is a form of vector capable of expressing the polynucleotide. A polynucleotide sequence is 'operably linked' to a regulatory sequence when the regulatory sequence affects the expression (e.g., level, timing, or location of expression) of the polynucleotide sequence. The regulatory sequence is a sequence that affects the expression (e.g., level, timing, or location of expression) of the nucleic acid to which it is operably linked. The regulatory sequence may exert its effect directly on the regulated nucleic acid or through the action of one or more other molecules (e.g., polypeptides binding to the regulatory sequence and/or the nucleic acid). The regulatory sequence includes promoters, enhancers, and other expression control elements.

The cell line of the present invention is not particularly limited as long as it is a cell that can be used to express the polynucleotide encoding the antibody or fragment thereof included in the expression vector of the present invention. The host cell transformed with the expression vector according to the present invention may be a prokaryote (e.g., *Escherichia coli*)*,* a eukaryote (e.g., yeast or other fungi), a plant cell (e.g., tobacco or tomato plant cells), an animal cell (e.g., human cells, monkey cells, hamster cells, rat cells, mouse cells, insect cells), or a hybridoma derived therefrom. Preferably, it may be a cell derived from a mammal, including humans.

Prokaryotes suitable for this purpose include Gram-negative or Gram-positive organisms, such as *Enterobacteriaceae,* e.g., *Escherichia* (e.g., *E. coli*)*, Enterobacter, Erwinia, Klebsiella, Proteus, Salmonella* (e.g., *Salmonella typhimurium*), *Serratia* (e.g., *Serratia marcescans*), and *Shigella*; *Bacillus* belonging to *Bacilli* (e.g., B. *subtilis* and B. *licheniformis*); *Pseudomonas* (e.g., *P aeruginosa*); and *Streptomyces.* The cell of the present invention is not particularly limited as long as it can express the vector of the present invention, but preferably, it may be *E. coli.*

The eukaryote as the cell of the present invention is most commonly Saccharomyces cerevisiae. However, many other genera, species, and strains, for example, *Schizosaccharomyces pombe, Kluyveromyces* hosts, for example, *K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans,* and *K. marxianus; Yarrowia* (EP 402,226); *Pichia pastoris* (EP 183,070); *Candida; Trichoderma reesia* (EP 244,234); *Neurospora crassa; Schwanniomyces,* for example, *Schwanniomyces occidentalis;* and filamentous fungi, for example, *Neurospora, Penicillium, Tolypocladium,* and *Aspergillus* hosts, for example, *A. nidulans* and *A. niger,* can be used but not limited thereto.

The 'transformation' refers to the modification of the genotype of a host cell by the introduction of a foreign polynucleotide (a polynucleotide encoding the antibody or a fragment thereof of the present invention), and it means that the foreign polynucleotide has been introduced into the host cell regardless of the method used for the transformation. The foreign polynucleotide introduced into the host cell may be integrated and maintained in the genome of the host cell or may be maintained without integration, and the present invention includes both.

A recombinant vector capable of expressing the antibody or a fragment thereof of the present invention can be introduced into cells to transform them for producing the antibody or a fragment thereof by methods known in the art, for example, but not limited to, transient transfection, microinjection, transduction, cell fusion, calcium phosphate precipitation, liposome-mediated transfection, DEAE dextran-mediated transfection, polybrene-mediated transfection, electroporation, gene gun, and other known methods for introducing nucleic acids into cells.

In addition, the cell of the present invention is a cultured cell that can be transformed or transfected with the polynucleotide of the present invention or a vector comprising the same, which can subsequently be expressed in the host cell. A recombinant cell refers to a cell transformed or transfected with a polynucleotide to be expressed. The cell of the present invention may also include the polynucleotide of the present invention but may not express it at the desired level unless the regulatory sequence is operably linked to the polynucleotide within the cell.

The cell of the present invention can be cultured in various media. Commercially available media, such as Ham's F10 (Sigma-Aldrich Co., St. Louis, MO), Minimal Essential Medium (MEM, Sigma-Aldrich Co.), RPMI-1640 (Sigma-Aldrich Co.), and Dulbecco's Modified Eagle's Medium (DMEM, Sigma-Aldrich Co.), are suitable for culturing the cells. The media may, if necessary, be supplemented with hormones and/or other growth factors, salts, buffers, nucleotides, antibiotics, trace elements, and glucose or equivalent energy sources.

The present invention provides a method for producing an antibody or a fragment thereof that binds to human VISTA protein, comprising culturing the cell under conditions in which the polynucleotide is expressed to produce a polypeptide comprising a light chain and a heavy chain variable region, and recovering the polypeptide from the cell or the culture medium in which the cell was cultured.

The cell for the production method in the present invention is as described above and includes a polynucleotide encoding the antibody of the present invention. The polypeptide of the production method may be the antibody or a fragment thereof of the present invention itself, or it may be a polypeptide to which other amino acid sequences in addition to the antibody or a fragment thereof of the present invention are further bound.

In this case, the additional amino acid sequences can be removed from the antibody or a fragment thereof of the present invention using methods well known to those skilled in the art. The culture conditions, including the composition of the medium, may vary depending on the type of cell, and these can be appropriately selected and adjusted by those skilled in the art.

The antibody molecule may accumulate in the cytoplasm of the cell, be secreted from the cell, or be targeted to the periplasm or extracellular medium (supernatant) by an appropriate signal sequence, and it is preferable that it is targeted to the periplasm or extracellular medium. Additionally, it is preferable to refold the produced antibody molecule using methods well known to those skilled in the art to have a functional conformation. The recovery of the polypeptide may vary depending on the characteristics of the produced polypeptide and the cell, and these can be appropriately selected and adjusted by those skilled in the art.

The polypeptide may be produced intracellularly, in the surrounding cytoplasmic space, or directly secreted into the medium. If the polypeptide is produced intracellularly, the cell may be disrupted as a first step to release the protein. Particulate debris, host cells, or lysed fragments may be removed, for example, by centrifugation or ultrafiltration. If the antibody is secreted into the medium, the supernatant from such expression systems is typically first concentrated using commercially available protein concentration filters, such as Amicon or Millipore Pellicon ultrafiltration units. Protease inhibitors, such as PMSF, may be included in any preceding step to inhibit protein degradation, and antibiotics may be included to prevent the growth of accidental contaminants. Antibodies produced from cells may be purified, for example, using hydroxyapatite chromatography, gel electrophoresis, dialysis, and affinity chromatography, and the antibody of the present invention is preferably purified through affinity chromatography.

According to one embodiment of the present invention, the antibody of the present invention has been confirmed to have excellent binding neutralization ability against PSGL1 and VSIG3.

In addition, according to another embodiment of the present invention, the antibody of the present invention was confirmed to have strong ADCC (Antibody Dependent Cellular Cytotoxicity) efficacy in a concentration-dependent manner when tested against NIH/3T3-VISTA cell lines using NK92MI-CD16 cell lines.

Furthermore, according to another embodiment of the present invention, the antibody of the present invention was confirmed to activate immunity in a concentration-dependent manner.

These results suggest that the antibody of the present invention specifically binds to the VISTA protein, thereby activating immune cells in the body and consequently killing cancer cells. In fact, when the antibody according to the present invention was administered to mice induced with colorectal cancer, the tumor size was significantly reduced compared to the control group administered with IgG.

Therefore, the present invention also provides a pharmaceutical composition for preventing or treating cancer comprising the antibody or a fragment thereof as an active ingredient.

In the present invention, the cancer may be a solid cancer or a non-solid cancer. Solid cancer refers to cancer tumors occurring in organs such as the liver, lungs, breasts, and skin. Non-solid cancer refers to cancer occurring in the blood and is also called blood cancer. The cancer may be carcinoma, sarcoma, cancer derived from hematopoietic cells, germ cell tumor, or blastoma. The cancer may be selected from the group consisting of, for example, breast cancer, colorectal cancer, head and neck cancer, colon cancer, skin cancer, pancreatic cancer, lung cancer, gastric cancer, ovarian cancer, prostate cancer, bladder cancer, urethral cancer, liver cancer, kidney cancer, clear cell sarcoma, melanoma, brain and spinal cord tumor, brain cancer, thymoma, mesothelioma, esophageal cancer, bile duct cancer, testicular cancer, germ cell tumor, thyroid cancer, parathyroid cancer, cervical cancer, endometrial cancer, lymphoma, myelodysplastic syndromes (MDS), myelofibrosis, acute leukemia, chronic leukemia, multiple myeloma, Hodgkin's disease, endocrine cancer, and sarcoma.

The composition according to the present invention may consist of only the antibody or fragment thereof according to the present invention, or may be formulated in a suitable form together with a pharmaceutically acceptable carrier, and may further comprise excipients or diluents. The term "pharmaceutically acceptable" as used herein refers to a physiologically acceptable and non-toxic composition that does not cause allergic reactions such as gastrointestinal disturbances, dizziness, or similar reactions when administered to humans. The carrier may include all types of solvents, dispersion media, water-in-oil or oil-in-water emulsions, aqueous compositions, liposomes, microbeads, and microemulsions.

The pharmaceutically acceptable carrier may further include, for example, carriers for oral administration or carriers for parenteral administration. Carriers for oral administration may include lactose, starch, cellulose derivatives, magnesium stearate, stearic acid, and the like. Additionally, it may include various drug delivery substances used for oral administration of peptide formulations. Furthermore, carriers for parenteral administration may include water, suitable oils, saline, aqueous glucose, and glycols, and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol. The pharmaceutical composition of the present invention may further comprise lubricants, wetting agents, sweeteners, flavoring agents, emulsifiers, suspending agents, and the like. Other pharmaceutically acceptable carriers and formulations may be referred to in known literature.

The composition of the present invention can be administered to mammals, including humans, by any method. For example, it can be administered orally or parenterally. Parenteral administration methods may include, but are not limited to, intravenous, intramuscular, intra-arterial, intraosseous, intradural, intracardiac, transdermal, subcutaneous, intraperitoneal, intranasal, enteral, topical, sublingual, or rectal administration.

The pharmaceutical composition of the present invention can be formulated as an oral or parenteral preparation according to the administration routes described above. In the case of oral preparations, the composition of the present invention can be formulated into powders, granules, tablets, pills, sugar-coated tablets, capsules, liquids, gels, syrups, slurries, suspensions, and the like using methods known in the art. For example, oral preparations can be obtained by mixing the active ingredient with a solid excipient, grinding it, adding suitable auxiliary agents, and processing it into a granulated mixture to produce tablets or sugar-coated tablets. Examples of suitable excipients include sugars such as lactose, dextrose, sucrose, sorbitol, mannitol, xylitol, erythritol, and maltitol; starches such as corn starch, wheat starch, rice starch, and potato starch; celluloses such as cellulose, methyl cellulose, sodium carboxymethyl cellulose, and hydroxypropyl methylcellulose; and fillers such as gelatin and polyvinylpyrrolidone. Additionally, disintegrants such as cross-linked polyvinylpyrrolidone, agar, alginic acid, or sodium alginate may be added as needed. Furthermore, the pharmaceutical composition of the present invention may further comprise anti-coagulants, lubricants, wetting agents, flavoring agents, emulsifiers, and preservatives. In the case of parenteral preparations, they can be formulated into injections, creams, lotions, topical ointments, oils, moisturizers, gels, aerosols, and nasal inhalants using methods known in the art. These formulations are described in generally known literature in the field of pharmaceutical chemistry.

The total effective amount of the composition of the present invention can be administered to a patient as a single dose or by a fractionated treatment protocol involving multiple doses over a long period. The pharmaceutical composition of the present invention may vary the content of the active ingredient depending on the severity of the disease. Preferably, the total preferred dose of the pharmaceutical composition of the present invention is about 0.01 µg to 10,000 mg per kg of patient body weight per day, and most preferably 0.1 µg to 500 mg. However, the dosage of the pharmaceutical composition may be determined by considering various factors such as the formulation method, administration route, frequency of treatment, as well as the patient's age, weight, health condition, gender, severity of the disease, diet, and excretion rate. Considering these factors, a person skilled in the art will be able to determine the appropriate effective dosage of the composition of the present invention. The pharmaceutical composition according to the present invention is not particularly limited in its formulation, administration route, or administration method as long as it exhibits the effects of the present invention.

Additionally, the present invention provides the use of the antibody or fragment thereof for manufacturing a pharmaceutical composition for treating cancer.

Furthermore, the present invention provides a method for treating cancer comprising administering an effective amount of a pharmaceutical composition comprising the antibody or fragment thereof as an active ingredient to a subject in need thereof.

The term "effective amount" as used in the present invention refers to an amount that, when administered to a subject, exhibits effects such as improvement, treatment, detection, diagnosis, inhibition, or reduction of cancer or cancer-related conditions. The term "subject" may include animals, preferably mammals, particularly humans, and may also include cells, tissues, or organs derived from animals. The subject may be a patient in need of the aforementioned effects.

The term "treatment" as used in the present invention comprehensively refers to improving symptoms caused by cancer or the aforementioned conditions, and may include curing, substantially preventing, or improving the condition. It may also include alleviating, curing, or preventing one or more symptoms or most symptoms caused by the condition, but is not limited thereto.

The term "comprising" as used herein is intended to have the same meaning as "including" or "characterized by" and does not exclude additional components or steps not specifically mentioned in the composition or method according to the present invention. The term "consisting of" means excluding additional elements, steps, or components not specifically described. The term "consisting essentially of" means that the composition or method may include substances or steps that do not substantially affect the basic characteristics of the described substances or steps.

### Effect of the Invention

Accordingly, the present invention provides an antibody or a fragment thereof that binds to human VISTA protein. The antibody of the present invention binds to the VISTA protein of T cells, thereby inhibiting the inactivation of T cells by cancer cells, enabling T cells to attack cancer cells, and at the same time, exerting a strong ADCC effect through NK cells, thus exhibiting remarkable effects as an anticancer agent.

### Brief Description of the Drawings

Figure 1 shows that the top 6 clones among the scFv sequences obtained through phage display were converted into the form of Full IgG1, and the clones were expressed and purified as confirmed by SDS-PAGE.
Figure 2 shows the results of the binding ability of the anti-VISTA antibody using a flow cytometer with the NIH/3T3 cell line.
Figure 3 shows the results of a Western blot using the NIH/3T3 cell line overexpressing human VISTA protein and the anti-VISTA antibody.
Figure 4 shows the results of immunoprecipitation (IP) using the NIH/3T3 cell line overexpressing human VISTA protein and the anti-VISTA antibody.
Figure 5 shows the results of the human-mouse cross-reactivity of the anti-VISTA antibody using ELISA.
Figure 6 shows the results of the change in binding ability of the anti-VISTA antibody according to pH changes using a flow cytometer.
Figure 7 shows the results of the binding neutralization ability against VSIG3, which binds to VISTA, at neutral pH (pH 7.0), through competitive ELISA.
Figure 8 shows the results of the binding neutralization ability against PSGL1, which binds to VISTA, at acidic pH (pH 6.0), through competitive ELISA.
Figure 9 shows the results of the binding neutralization ability against PSGL1, which binds to VISTA, at acidic pH (pH 6.0), using a flow cytometer.
Figure 10 shows the results of an ADCC assay using the NK92MI-CD16 cell line on the NIH/3T3 cell line overexpressing human VISTA protein.
Figure 11 shows the results of co-culturing CD14 monocytes and CD4 T cells, which were separated and activated from human peripheral blood mononuclear cells, with the anti-VISTA antibody.
Figure 12 shows the ELISA results for the anti-VISTA antibody and anti-PD-1 antibody after stimulating human peripheral blood mononuclear cells with Staphylococcal enterotoxin B (SEB).
Figure 13 shows the ELISA results (INF-γ) after stimulating human peripheral blood mononuclear cells with Staphylococcal enterotoxin B (SEB), treating them with VISTA protein, and then treating them with the anti-VISTA antibody.
Figure 14 shows the ELISA results (TNF-α) after stimulating human peripheral blood mononuclear cells with Staphylococcal enterotoxin B (SEB), treating them with VISTA protein, and then treating them with the anti-VISTA antibody.
Figure 15 shows the ELISA results (IL-6) after stimulating human peripheral blood mononuclear cells with Staphylococcal enterotoxin B (SEB), treating them with VISTA protein, and then treating them with the anti-VISTA antibody.
Figure 16 shows the ELISA results (IL-6) after conducting a mixed lymphocyte reaction using human peripheral blood mononuclear cells from different donors and then treating them with the anti-VISTA antibody.
Figure 17 shows the results of *in vivo* anti-cancer efficacy of the anti-VISTA antibody using a mouse model introduced with human VISTA protein.

Hereinafter, the present invention will be described in detail.

However, the following examples are merely illustrative of the present invention, and the scope of the present invention is not limited to the following examples.

### Example 1. Screening of scFv specifically binding to human VISTA protein

### 1-1. Antigen and scFv phage selection

As an antigen, VISTA was provided in the form of a recombinant protein (hVISTA-Fc, RND systems, Cat. No 7126-B7) during the screening process. To screen for antibodies specifically binding to VISTA, a phage display method (phage library display) was used. The library used was a synthetic human scFv library, and specific information about the library is described in publicly known literature in the art. The scFv expressed from the scFv library was tagged with an HA tag to allow detection by an anti-HA FITC antibody (Genscript, A01621). Using the scFv library, biopanning was performed as follows. The scFv library stock was blocked at room temperature with 3% Skim milk/PBS. The antigen (hVISTA-Fc, RND systems, Cat. No 7126-B7) was added to an immunotube at a concentration of 15 µg/mL in 1 mL and coated at 200 rpm, 4°C for more than 12 hours. The coated immunotube was washed three times with 0.05% PBS-T and blocked with 1 mL of 6% Skim milk blocking buffer at room temperature for 2 hours. After 2 hours, it was washed three times with 0.05% PBS-T. The blocked scFv library stock was added to the antigen-coated immunotube and incubated at 200 rpm, 37°C for 1 hour. The unbound scFv library stock was discarded, and the tube was washed three times with 0.05% PBS-T. To elute specifically bound scFv-phages, they were reacted with 100 mM TEA (triethylamine) at room temperature for 5 minutes, neutralized with pH 8.5 Tris, and prepared in the form of an scFv-antigen conjugate. The prepared scFv-antigen conjugate was added to *E. coli* TG1 cells for infection, and the cells were cultured overnight at 37°C on LB/ampicillin/glucose agar medium. The *E. coli* TG1 cells were transferred to SB/ampicillin medium and cultured until the OD600 value reached 0.5, after which 1x10¹¹~1x10¹² helper phages were added and cultured again at 37°C for 1 hour. Kanamycin was then added, and the culture was incubated overnight again. The overnight culture was centrifuged, and the supernatant was reacted with a PEG solution at 4°C, followed by centrifugation to separate the pellet. The pellet was dissolved in PBS, centrifuged, and the resulting supernatant was secured as the scFv library solution. This process was repeated four times to secure scFv candidates specifically binding to the VISTA antigen.

### 1-2. Selection of scFv antibodies specifically binding to VISTA

To select scFv with excellent binding ability from the scFv candidates obtained in Example 1-1, ELISA analysis was performed on VISTA-expressing cell lines. The VISTA-expressing cell line was prepared by transfecting a human VISTA expression vector into NIH/3T3 cells, followed by treatment with 500 µg/mL of Hygromycin B to select transformants. Each library stock obtained after panning at each stage in Example 1-1 was cultured overnight on SB/ampicillin/glucose agar medium, and each single colony was inoculated into 200 µL of SB/ampicillin medium and cultured at 37°C for 3 hours. Subsequently, IPTG was added to a final concentration of 1 mM, and the culture was incubated again at 30°C overnight. After the culture was completed, the culture medium was centrifuged to separate the cells, which were then lysed using TES buffer to obtain scFv. The obtained scFv was treated on a plate where VISTA-expressing cells were dispensed at 1x10⁵ per well and reacted at room temperature for 1 hour. Then, a secondary antibody (anti-HA HRP, santacruz, Cat. NO. sc-7392) was added and reacted for 40 minutes. After the secondary antibody reaction was completed, TMB was added to induce a color reaction, and the results were analyzed using an ELISA reader (450 nm). By relatively comparing the ELISA analysis values, the top six excellent scFv were selected (4D2, 4F2, 4G3, 4H2, 4A2, 4A3).

The amino acid sequences of the selected scFv are shown in Table 1 below.

**Table 1**

| Antibody | | | Amino acid sequence | SEQ ID NO. |
|---|---|---|---|---|
| 4D2 | Heavy chain variable region | CDR1 | SYWIA | SEQ ID NO.1 |
| | | CDR2 | TISGSGGYTYYAESVKG | SEQ ID NO.2 |
| | | CDR3 | RDSSGWLNY | SEQ ID NO.3 |
| | Light chain variable region | CDR1 | RASQSITSHLA | SEQ ID NO.4 |
| | | CDR2 | DASNRAT | SEQ ID NO.5 |
| | | CDR3 | QQYNNWPPIT | SEQ ID NO.6 |
| 4F2 | Heavy chain variable region | CDR1 | DYYMH | SEQ ID NO.9 |
| | | CDR2 | EIIPIFDTANYAQKFQD | SEQ ID NO.10 |
| | | CDR3 | RSTGDYFDY | SEQ ID NO.11 |
| | Light chain variable region | CDR1 | SGDKLGNKYTS | SEQ ID NO.12 |
| | | CDR2 | DNNKRPS | SEQ ID NO.13 |
| | | CDR3 | GTWDSSLSAYV | SEQ ID NO.14 |
| 4G3 | Heavy chain variable region | CDR1 | EKSIH | SEQ ID NO.17 |
| | | CDR2 | GTSANTGNTYYADSVKG | SEQ ID NO.18 |
| | | CDR3 | HHNWAGYFDY | SEQ ID NO.19 |
| | Light chain variable region | CDR1 | QASHAISNYIN | SEQ ID NO.20 |
| | | CDR2 | DASNRAT | SEQ ID NO.21 |
| | | CDR3 | QQSNSFPYT | SEQ ID NO.22 |
| 4H2 | Heavy chain variable region | CDR1 | TAWMS | SEQ ID NO.25 |
| | | CDR2 | RVNPSDSHTDYSPSFQD | SEQ ID NO.26 |
| | | CDR3 | RVPPGYGFDI | SEQ ID NO.27 |
| | Light chain variable region | CDR1 | QASQDITNYLN | SEQ ID NO.28 |
| | | CDR2 | DASNRAT | SEQ ID NO.29 |
| | | CDR3 | QQSYSTPFT | SEQ ID NO.30 |
| 4A2 | Heavy chain variable region | CDR1 | NYAMS | SEQ ID NO.33 |
| | | CDR2 | EISHSGTTNYNPSLKS | SEQ ID NO.34 |
| | | CDR3 | TRAIWGFDY | SEQ ID NO.35 |
| | Light chain variable region | CDR1 | SRENIGSRSVH | SEQ ID NO.36 |
| | | CDR2 | DNNERAS | SEQ ID NO.37 |
| | | CDR3 | QAWDSSTAV | SEQ ID NO.38 |
| 4A3 | Heavy chain variable region | CDR1 | NYYMH | SEQ ID NO.41 |
| | | CDR2 | SISFTGSTSYNPSLKS | SEQ ID NO.42 |
| | | CDR3 | RLGIPFFTYLDY | SEQ ID NO.43 |
| | Light chain variable region | CDR1 | GGNNIGSKSVH | SEQ ID NO.44 |
| | | CDR2 | EDTERPS | SEQ ID NO.45 |
| | | CDR3 | QAWDSSTVV | SEQ ID NO.46 |

### Example 2. Conversion, expression, and purification of scFv antibodies into IgG

### 2-1. Preparation of full IgG expression vector

The selected scFv was converted into the form of IgG, a more commonly used antibody. Based on the CDR region of the scFv, an expression vector capable of expressing the full IgG form was prepared. First, the light chain variable region and heavy chain variable region of the scFv were obtained through PCR, using the primers shown in Table 2 below. The light chain variable region sequence was cloned into the expression vector pcDNA 3.3 (Invitrogen), which contains the light chain constant region sequence, and the heavy chain variable region sequence was cloned into the expression vector pOptiVEC (Invitrogen), which contains the heavy chain constant region sequence, respectively. Together with the light chain and heavy chain constant regions cloned from the vectors, the light chain variable region and heavy chain variable region of the scFv were expressed, resulting in the production of a full IgG antibody containing the CDR region of the scFv.

**Table 2**

| Antibody | Variable region | Primer sequence | SEQ ID NO. |
|---|---|---|---|
| 4D2 | Light chain F | ATT CGA TCG ATA TGG AGA CAG ACA CAC TCC TGC TAT GGG TAC TGC TGC TCT GGG TTC CAG GTT CCA CGT GGG AAA TCG TGC TGA CCC AGA GC | SEQ ID NO.49 |
| | Light chain R | AGC CAC CGT ACG TTT GAT TTC CAC TTT AGT GCC | SEQ ID NO.50 |
| | Heavy chain F | ATT CGA TCG ATA TGG AGA CAG ACA CAC TCC TGC TAT GGG TAC TGC TGC TCT GGG TTC CAG GTT CCA CGT GGG AAG TGC AGC TGC TGG AAA GT | SEQ ID NO.51 |
| | Heavy chain R | CTT GGT GCT AGC GCT GCT CAC GGT CAC CAG AGT | SEQ ID NO.52 |
| 4F2 | Light chain F | ATT CGA TCG ATA TGG AGA CAG ACA CAC TCC TGC TAT GGG TAC TGC TGC TCT GGG TTC CAG GTT CCA CGT GGG AAA TCG TGC TGA CCC AGA GC | SEQ ID NO.53 |
| | Light chain R | AGC CAC CGT ACG TTT GAT TTC CAC TTT AGT GCC | SEQ ID NO.54 |
| | Heavy chain F | ATT CGA TCG ATA TGG AGA CAG ACA CAC TCC TGC TAT GGG TAC TGC TGC TCT GGG TTC CAG GTT CCA CGT GGG AAG TGC AGC TGC TGG AAA GT | SEQ ID NO.55 |
| | Heavy chain R | CTT GGT GCT AGC GCT GCT CAC GGT CAC CAG AGT | SEQ ID NO.56 |
| 4G3 | Light chain F | ATT CGA TCG ATA TGG AGA CAG ACA CAC TCC TGC TAT GGG TAC TGC TGC TCT GGG TTC CAG GTT CCA CGT GGG AAA TCG TGC TGA CCC AGA GC | SEQ ID NO.57 |
| | Light chain R | AGC CAC CGT ACG TTT GAT TTC CAC TTT AGT GCC | SEQ ID NO.58 |
| | Heavy chain F | ATT CGA TCG ATA TGG AGA CAG ACA CAC TCC TGC TAT GGG TAC TGC TGC TCT GGG TTC CAG GTT CCA CGT GGG AAG TGC AGC TGC TGG AAA GT | SEQ ID NO.59 |
| | Heavy chain R | CTT GGT GCT AGC GCT GCT CAC GGT CAC CAG AGT | SEQ ID NO.60 |
| 4H2 | Light chain F | ATT CGA TCG ATA TGG AGA CAG ACA CAC TCC TGC TAT GGG TAC TGC TGC TCT GGG TTC CAG GTT CCA CGT GGG AAA TCG TGC TGA CCC AGA GC | SEQ ID NO.61 |
| | Light chain R | AGC CAC CGT ACG TTT GAT TTC CAC TTT AGT GCC | SEQ ID NO.62 |
| | Heavy chain F | ATT CGA TCG ATA TGG AGA CAG ACA CAC TCC TGC TAT GGG TAC TGC TGC TCT GGG TTC CAG GTT CCA CGT GGG AAG TGC AGC TGC TGG AAA GT | SEQ ID NO.63 |
| | Heavy chain R | CTT GGT GCT AGC GCT GCT CAC GGT CAC CAG AGT | SEQ ID NO.64 |
| 4A2 | Light chain F | ATT CGA TCG ATA TGG AGA CAG ACA CAC TCC TGC TAT GGG TAC TGC TGC TCT GGG TTC CAG GTT CCA CGT GGG AAA TCG TGC TGA CCC AGA GC | SEQ ID NO.65 |
| | Light chain R | AGC CAC CGT ACG TTT GAT TTC CAC TTT AGT GCC | SEQ ID NO.66 |
| | Heavy chain F | ATT CGA TCG ATA TGG AGA CAG ACA CAC TCC TGC TAT GGG TAC TGC TGC TCT GGG TTC CAG GTT CCA CGT GGG AAG TGC AGC TGC TGG AAA GT | SEQ ID NO.67 |
| | Heavy chain R | CTT GGT GCT AGC GCT GCT CAC GGT CAC CAG AGT | SEQ ID NO.68 |
| 4A3 | Light chain F | ATT CGA TCG ATA TGG AGA CAG ACA CAC TCC TGC TAT GGG TAC TGC TGC TCT GGG TTC CAG GTT CCA CGT GGG AAA TCG TGC TGA CCC AGA GC | SEQ ID NO.69 |
| | Light chain R | AGC CAC CGT ACG TTT GAT TTC CAC TTT AGT GCC | SEQ ID NO.70 |
| | Heavy chain F | ATT CGA TCG ATA TGG AGA CAG ACA CAC TCC TGC TAT GGG TAC TGC TGC TCT GGG TTC CAG GTT CCA CGT GGG AAG TGC AGC TGC TGG AAA GT | SEQ ID NO.71 |
| | Heavy chain R | CTT GGT GCT AGC GCT GCT CAC GGT CAC CAG AGT | SEQ ID NO.72 |

### 2-2. Preparation of full IgG antibody-expressing cell line

Using CHO-S cells (Life Technologies Inc.), IgG antibody-expressing cell lines were prepared. The gene sequences coding for the heavy and light chains obtained in Example 2-1 were codon-optimized for *Cricetulus griseus* and cloned into the Freedom^{®} pCHO 1.0 Vector. The vector was transfected into CHO-S cells using a transfection reagent (FreeStyle^{™} MAX Reagent; Life Technologies Inc.). To select antibody-expressing cell lines after transfection, a two-step selection process was performed using puromycin and MTX (methotrexate). Specifically, in the first selection, puromycin 10 µg/mL and MTX 100 nM or puromycin 20 µg/mL and MTX 200 nM were used, and when the cell survival rate met the criteria, the second selection process was performed. In the second selection, puromycin 30 µg/mL and MTX 500 nM or puromycin 50 µg/mL and MTX 1000 nM were used, and when the final cell survival rate met the criteria, the second selection was completed, and groups with high expression levels were selected through SFB (Simple Fed Batch).

### 2-3. Production and purification of full IgG antibody

Each antibody-producing cell line prepared in Example 2-2 was cultured in CD FortiCHO^{™} medium under conditions of 8% CO₂, 37°C, 100~120 rpm, with glucose added at 4 g/L, 4 g/L, and 6 g/L on days 3, 5, and 7, respectively, for a total of 14 days. After the culture was completed, the culture medium was centrifuged at 6000g using an ultra-centrifuge, and the supernatant was filtered using a 0.2 µm filter. For purification, Protein A resin (Mabselect SuRe, 11-0026-01 AD, GE Healthcare Life Sciences) was used, along with an equilibration buffer (20 mM Sodium Phosphate, 150 mM NaCl, pH 7.2), a wash buffer (35 mM Sodium Phosphate, 500 mM NaCl, pH 7.2), and an elution buffer (0.1M Sodium Citrate, pH 3.6). Using AKTA^{™} avant, the equilibration buffer was applied at twice the column volume, the wash buffer at five times the column volume, and the elution buffer at five times the column volume for purification. During elution, pH 8.0 Tris-HCl solution was added at 1/5 volume for neutralization. After two buffer exchanges with PBS using a filtration membrane (CelluSep, 1430-45), the solution was concentrated using a centrifugal filter (Amicon Ultra-15, UFC905024, Merck).

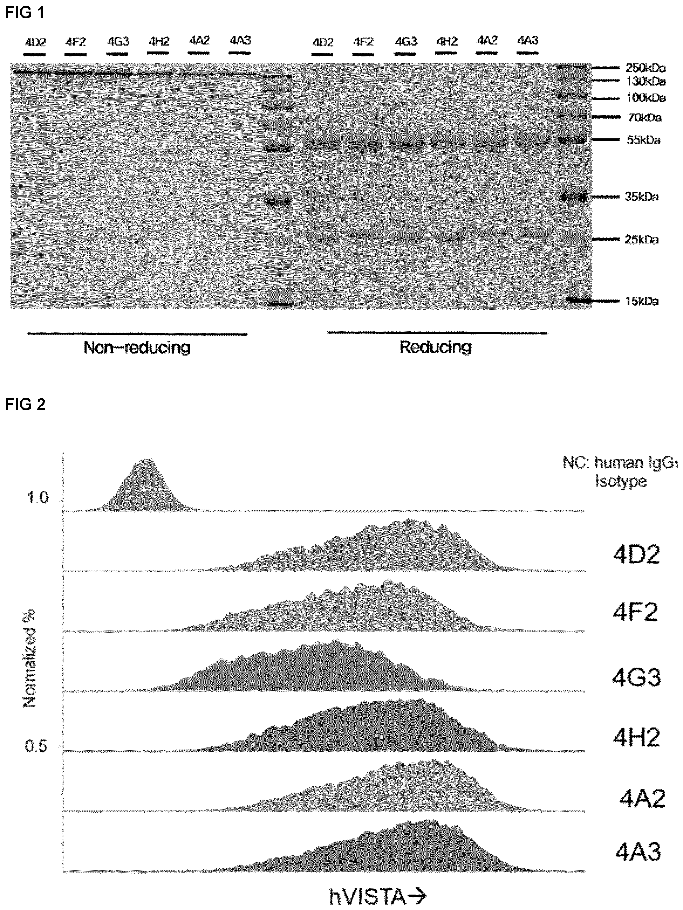

The IgG antibody proteins produced under both reducing and non-reducing conditions were confirmed using conventional SDS-PAGE techniques, and it was verified that the light and heavy chains of each antibody were well expressed at the expected molecular weights. Figure 1 shows the SDS-PAGE results for each IgG antibody.

### Example 3. Evaluation of binding specificity and binding affinity of the antibody to VISTA

### 3-1. Preparation of VISTA/NIH/3T3 cell line

To confirm the antigen specificity of the antibodies prepared in Example 2-3, the binding to human VISTA protein was evaluated.

The gene coding for VISTA was cloned into pCMV3-C-FLAG (Sino Biological). Each of the VISTA expression vectors thus produced was transfected into NIH/3T3 using the Fugene HD (E231A, Promega) transfection reagent, and hygromycin B was used to select resistant cell lines.

### 3-2. Evaluation of the binding ability of the antibody of the present invention to the VISTA/NIH/3T3 cell line

For the VISTA/NIH/3T3 cell line produced in Example 3-1, the cross-reactivity of the antibody prepared in Example 2-3 was confirmed. The naive NIH/3T3 cells were used as a negative control. First, the cells were dissociated into single cells using a cell dissociation buffer (Gibco, 13151-014), seeded at 2.5x10⁵ cells per well, and reacted with each antibody at 10 µg/ml on ice for 1 hour. After the reaction, the cells were washed with 1% FBS/PBS, treated with a secondary antibody, mouse anti-human IgG-PE (366904, Biolegend), at a 1:100 dilution, and reacted on ice for 1 hour. After the reaction, the cells were washed with 1% FBS/PBS and analyzed using a flow cytometer, BD FACS Lyrics.

Figure 2 shows the results of the flow cytometry analysis, which comparatively confirm the binding ability of the antibody to VISTA.

### 3-3. Confirmation of VISTA-specific binding - western blot

For the VISTA/NIH/3T3 cell line produced in Example 3-1, the ability of the antibody prepared in Example 2-3 to recognize denatured VISTA antigens was confirmed. The original NIH/3T3 cells were used as a negative control. First, the cells were dissociated into single cells using a cell dissociation buffer, lysed with RIPA buffer (BIOSESANG) containing protease inhibitors and phosphatase inhibitors at 200 rpm and 4°C for 1 hour. The lysate was centrifuged at 15,000 rpm and 4°C for 15 minutes, and the supernatant was collected. The dissolved proteins were quantified using a BCA assay (ThermoFisher Scientific). DTT-containing 5X sample loading buffer (BIOSESANG) was added to the quantified proteins, boiled for 10 minutes to reduce all proteins, and loaded same weight of proteins onto SDS-PAGE to separate proteins by size. The proteins were transferred to a PVDF membrane, blocked with 5% skim milk at 4°C for 1 hour, and incubated overnight with the antibody prepared in Example 2-3 as the primary antibody. After washing three times with 0.05% TBS-T, the membrane was treated with the secondary antibody, anti-human Fc HRP antibody (Genscript), at a 1:2000 dilution and incubated for 30 minutes. After washing three times with 0.05% TBS-T, ECL solution (Bio-rad) was added, and the membrane was developed on film in a darkroom.

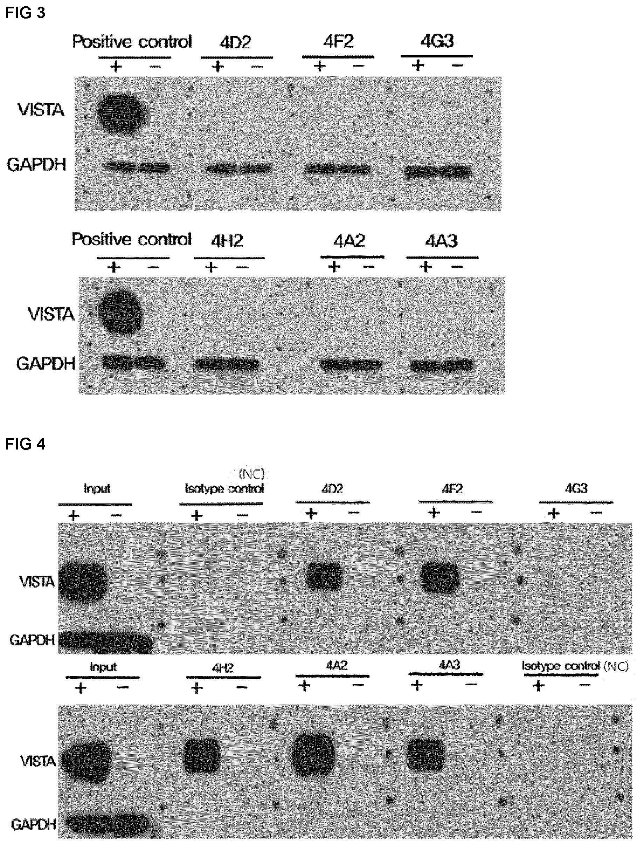

As shown in Figure 3, it was confirmed that the antibody did not recognize the denatured VISTA antigen.

### 3-4. Confirmation of VISTA-specific binding - immunoprecipitation

For the VISTA/NIH/3T3 cell line produced in Example 3-1, the ability of the antibody prepared in Example 2-3 to recognize native VISTA antigens was confirmed. The naive NIH/3T3 cells were used as a negative control. The cells were detached using a scraper and resuspended in PBS containing protease inhibitors and phosphatase inhibitors. The cells were physically lysed using a sonicator, and the proteins were quantified using a BCA assay. The quantified proteins were incubated with the antibody for 1 hour, while Protein A beads (Sigma) were blocked with 5% skim milk for 1 hour. The protein-antibody solution was then incubated with the blocked beads for 1 hour and washed three times with PBS. DTT-containing 5X sample loading buffer was added, and the mixture was incubated at 70°C for 10 minutes to dissociate and reduce the proteins and antibodies bound to the Protein A beads. The samples were subjected to western blot analysis, and the anti-human VISTA antibody (Cell Signaling Technology) was used to confirm whether the antibody recognized the native VISTA antigen.

As shown in Figure 4, it was confirmed that the antibody specifically binds to the native VISTA antigen.

### Example 4. Confirmation of human-mouse cross-reactivity of anti-VISTA antibody using ELISA

The human-mouse cross-reactivity of the antibody prepared in Example 2-3 was confirmed using ELISA. Mouse VISTA protein (mVISTA-Fc, R&D Systems, Cat. No. 7005-B7) and human VISTA protein (hVISTA-Fc, R&D Systems, Cat. No. 7126-B7) were each coated at 100 ng per well on a 96-well plate and incubated overnight at 4°C. The wells were washed three times with 0.05% PBS-T and blocked with 300 µl of 5% skim milk at room temperature for 2 hours. After blocking, the wells were washed three times with 0.05% PBS-T, and each antibody was added at 100 ng per well and incubated at room temperature for 1 hour. VSTB174 (J&J) and BMS767 (BMS) were used as positive controls in the same amount. After binding, the wells were washed three times with 0.05% PBS-T, treated with anti-human Fc HRP antibody (Genscript) at a 1:2000 dilution, and incubated for 30 minutes. After incubation, the wells were washed three times with 0.05% PBS-T, and 100 µl of TMB solution (Surmodics) was added to each well. The reaction was stopped after 20 minutes at room temperature by adding 50 µl of stop solution, and the absorbance at 450 nm was measured using an ELISA reader.

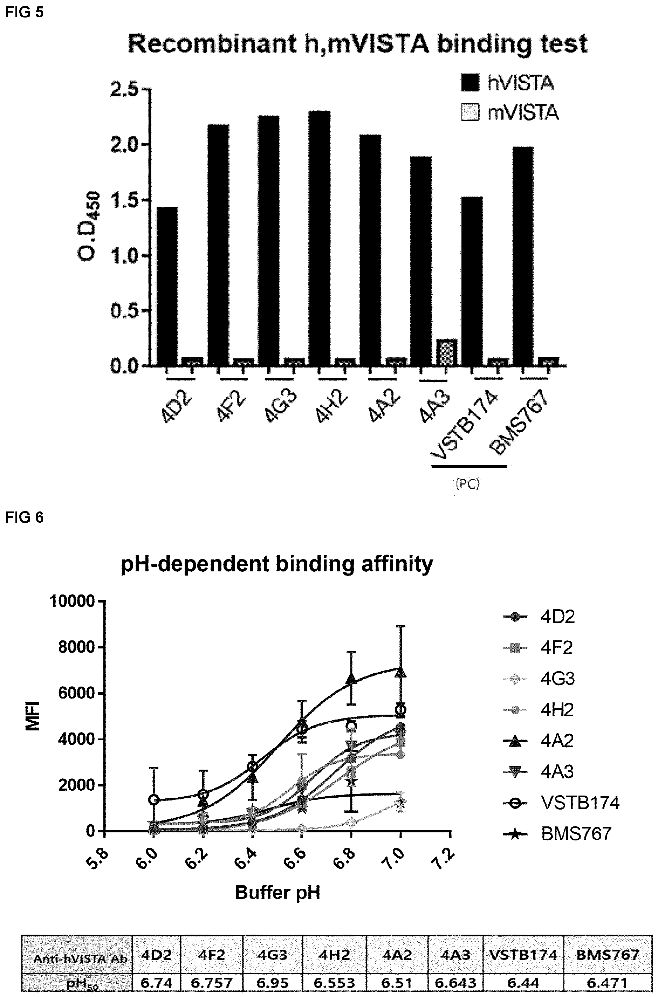

As shown in Figure 5, it was confirmed that the antibody did not recognize mouse VISTA but strongly bound to human VISTA.

### Example 5. Confirmation of pH-dependent binding ability and neutralization of VSIG3 and PSGL1 by anti-VISTA antibody

### Example 5-1. pH-dependent binding ability of anti-VISTA antibody

For the VISTA/NIH/3T3 cell line produced in Example 3-1, the pH-dependent VISTA antigen recognition ability of the antibody prepared in Example 2-3 was confirmed using flow cytometry. First, the cells were dissociated into single cells using a cell dissociation buffer and seeded at 2.5x10⁵ cells per well. Using an HCl-adjusted pH meter, six 1% BSA/PBS buffers were prepared with pH values ranging from 7.0 to 6.0. Each antibody was added at 10 µg/ml and reacted on ice for 1 hour. After the reaction, the cells were washed with 1% BSA/PBS buffer of the corresponding pH, treated with the secondary antibody, goat anti-human IgG-FITC (109-095-098, Jackson Immunoresearch), at a 1:100 dilution, and reacted on ice for 1 hour. After the reaction, the cells were washed with 1% BSA/PBS buffer of the corresponding pH and analyzed using a flow cytometer, BD FACS Lyrics.

Figure 6 shows the results of the flow cytometry analysis, which comparatively confirm the binding ability of the antibody to VISTA at different pH levels.

### Example 5-2. Neutralization of VSIG3 binding by anti-VISTA antibody

To confirm the neutralization ability of the antibody prepared in Example 2-3, the neutralization of VISTA-VSIG3 binding at pH 7.4 was confirmed using ELISA. Human VSIG3 protein (hVSIG3 Fc, R&D Systems, Cat. No. 9229-VS) was coated at 250 ng per well on a 96-well plate and incubated overnight at 4°C. The wells were washed three times with 0.1% PBS-T and blocked with 50 mg/mL BSA/PBS at room temperature for 2 hours. Meanwhile, human VISTA-biotin protein (hVISTA-Avi-Biotin, R&D Systems, Cat. No. AVI9057) at 100 nM was incubated with the anti-VISTA antibody diluted from 60 µg/ml in a 1:3 ratio at room temperature for 1 hour. The blocked 96-well plate was washed three times with 0.1% PBS-T, and 100 µl of the VISTA-antibody binding solution was added to each well and incubated at room temperature for 2 hours. VSTB174 (J&J) and BMS767 (BMS) were used as positive controls in the same amount. After binding, the wells were washed three times with 0.1% PBS-T, treated with Streptavidin-HRP (R&D Systems) at a 1:5000 dilution, and incubated at room temperature for 1 hour. After incubation, the wells were washed three times with 0.1% PBS-T, and 100 µl of TMB solution (Surmodics) was added to each well. The reaction was stopped after 20 minutes at room temperature by adding 50 µl of stop solution, and the absorbance at 450 nm was measured using an ELISA reader.

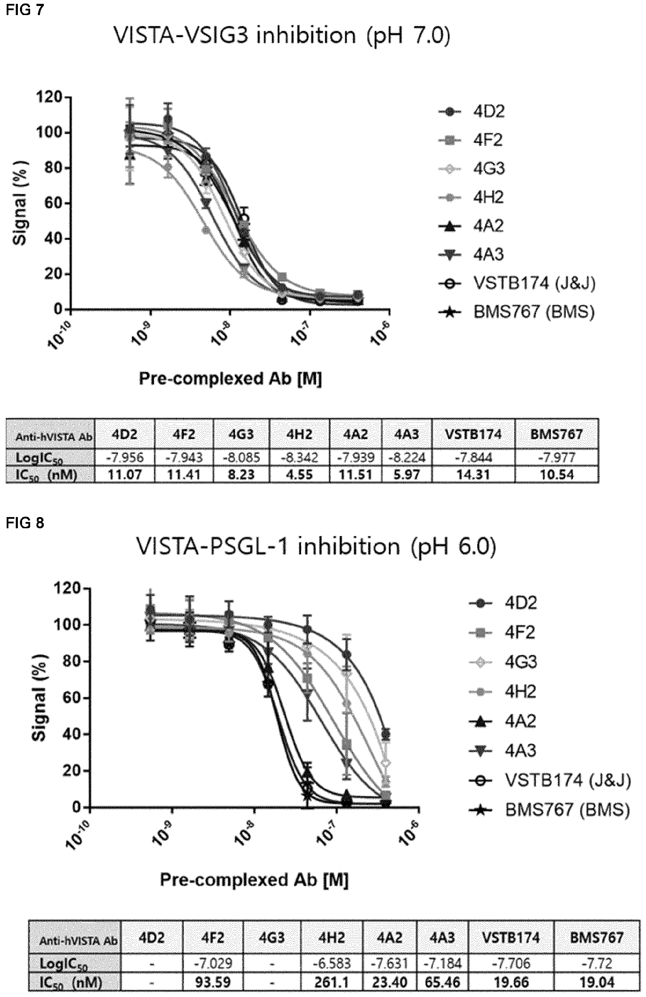

As shown in Figure 7, it was confirmed that the antibody inhibited the binding of VISTA to VSIG3 at neutral pH (pH 7.4).

### Example 5-3. Confirmation of the binding neutralization ability of anti-VISTA antibody to PSGL1 - ELISA

To confirm the binding neutralization ability of the antibody prepared in Example 2-3, the VISTA-PSGL1 binding neutralization ability at pH 6.0 was confirmed by ELISA. Human PSGL1 protein (hPSGL1 Fc, R&D systems, Cat. No 3345-PS) was added at 250 ng per well to a 96-well plate and coated overnight at 4°C. All subsequent processes were carried out by adjusting all buffers to pH 6.0 using HCl. The plate was washed three times with 0.1% PBS-T and blocked with 50 mg/mL BSA/PBS at room temperature for 2 hours. Meanwhile, human VISTA-biotin protein (hVISTA-Avi-Biotin, R&D systems, Cat. No. AVI9057) at 100 nM and anti-VISTA antibody diluted from 60 µg/mL at a 1/3 ratio were incubated at room temperature for 1 hour. The blocked 96-well plate was washed three times with 0.1% PBS-T, and 100 µL of the VISTA-antibody binding solution was added to each well and incubated at room temperature for 2 hours. As positive controls, equal amounts of VSTB174 (J&J) and BMS767 (BMS) were used. After binding, the plate was washed three times with 0.1% PBS-T, treated with Streptavidin-HRP (R&D systems) at a 1:5000 dilution, and incubated at room temperature for 1 hour. After incubation, the plate was washed three times with 0.1% PBS-T, and 100 µL of TMB solution (Surmodics) was added to each well and reacted at room temperature for 20 minutes. Then, 50 µL of stop solution was added, and the absorbance at 450 nm was measured using an ELISA reader.

As a result, as shown in Figure 8, it was confirmed that the antibody inhibited the binding of VISTA-PSGL1 at acidic pH (pH 6.0).

### Example 5-4. Confirmation of the binding neutralization ability of anti-VISTA antibody to PSGL1 - Flow cytometry

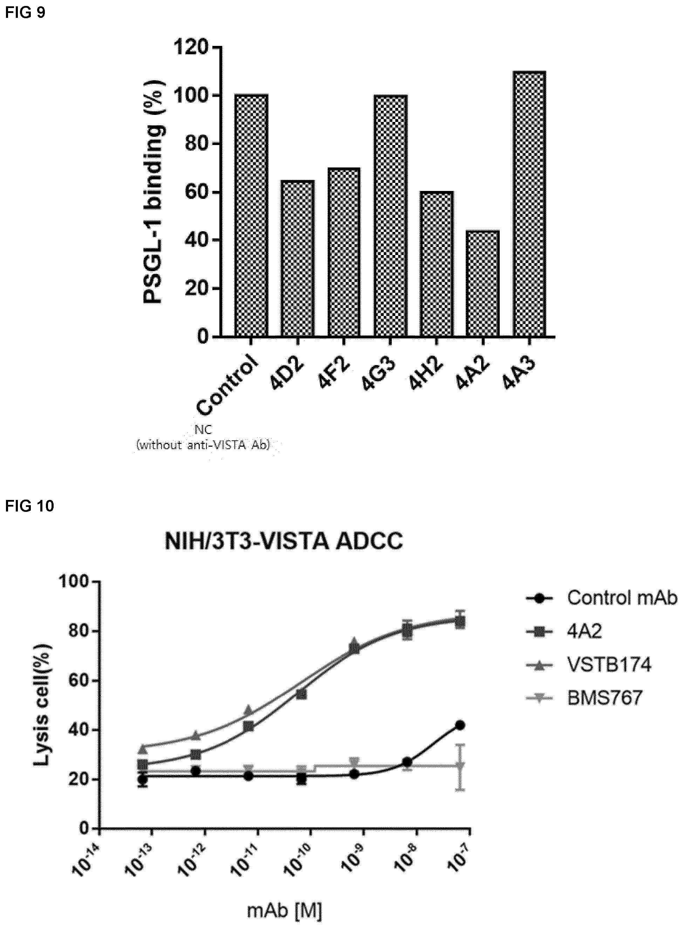

To confirm the binding neutralization ability of the antibody prepared in Example 2-3, the VISTA-PSGL1 binding neutralization ability at pH 6.0 was confirmed by flow cytometry. Using a Jurkat cell line expressing PSGL1, the Jurkat cell line and VISTA protein (R&D systems) were incubated with the anti-VISTA antibody in pH 6.0, 1% BSA/PBS buffer at 4°C for 30 minutes. Afterward, the cells were washed three times with the same buffer and incubated with the secondary antibody goat anti-human IgG-FITC (Jackson Immunoresearch) at 4°C for 30 minutes. The cells were then washed three times with the same buffer and analyzed using flow cytometer, BD FACS Lyrics. As a result, as shown in Figure 9, it was confirmed that the antibody inhibited the binding of PSGL1 expressed on Jurkat cells to VISTA protein at acidic pH (pH 6.0).

### Example 6. Confirmation of antibody-dependent cellular cytotoxicity

For the VISTA/NIH/3T3 cell line prepared in Example 3-1, the antibody-dependent cellular cytotoxicity of the antibody prepared in Example 2-3 was confirmed. The VISTA/NIH/3T3 cell line was used as the target cell, and a cell line transfected with human CD16 in the NK92MI cell line was used as the effector cell. The target cells were separated into single cells using a separation buffer, dispensed at 2.0x10⁴ cells per well into a 96-well plate, and incubated overnight. Effector cells were added at 1.0x10⁵ cells per well, and anti-VISTA antibody diluted from 10 µg/mL at a 1/10 ratio was added to the target cells and incubated at 37°C for 4 hours. Subsequently, 50 µL of the supernatant was used to perform an LDH assay (Promega, CytoTox 96^{®}) to confirm cytotoxicity.

As a result, as shown in Figure 10, it was confirmed that the antibody induced concentration-dependent cell death of cells expressing VISTA through antibody-dependent cellular cytotoxicity mediated by NK cells.

### Example 7. Confirmation of immune activation ability

### 7-1. Immune activation by anti-VISTA antibody (IFN-γ+CD4+)

To confirm the immune activation ability of the antibody prepared in Example 2-3, flow cytometry was performed using human immune cells. Peripheral blood concentrated in the LRS chamber was diluted with 2% FBS/PBS. Histopaque^{®}-1077 (Sigma) 25 mL was added to a 50 mL tube, and 25 mL of diluted peripheral blood was slowly layered on top, followed by centrifugation at 1200g for 10 minutes. After centrifugation, the supernatant was removed, and the human peripheral blood mononuclear cell (PBMC) layer was collected and transferred to a new 50 mL tube. The PBMCs were washed three times with 2% FBS/PBS washing buffer at 300g for 8 minutes to obtain PBMCs. From the obtained PBMCs, human CD4 T cells and human CD14 monocytes were separated using CD4 MicroBead (Miltenyi Biotec, Cat. No. 130-045-101) and CD14 MicroBead (Miltenyi Biotec, Cat. No. 130-050-201). For T cell activation, a 96-well plate was coated with anti-CD3 antibody (OKT3, Abcam) at a concentration of 2.5 µg/mL at room temperature for 2 hours, and the separated human CD4 T cells were added at 1.0x10⁵ cells per well. Human CD14 monocytes expressing VISTA were added at 5.0x10⁵ cells per well, along with 10 µg/mL of anti-VISTA antibody, and incubated at 37°C for 8 hours. To confirm interferon-gamma expressed inside CD4 T cells, Brefeldin A (Biolegend) was added, and mouse anti-human CD4 APC antibody (Biolegend) and mouse anti-human IFN-γ FITC antibody (Biolegend) were treated, followed by analysis using flow cytometer, BD FACS Lyrics.

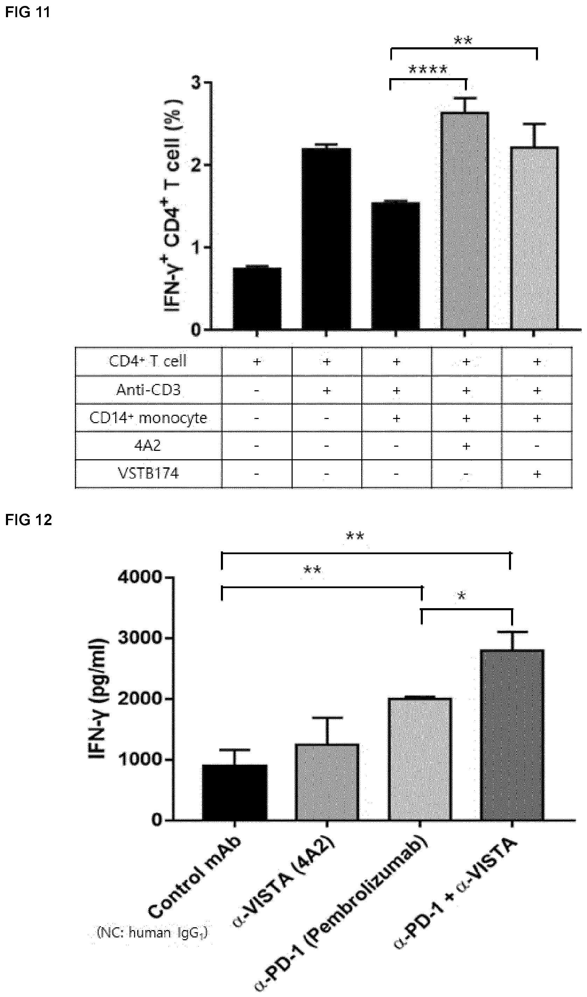

As a result, as shown in Figure 11, it was confirmed that the antibody restored IFN-γ⁺CD4⁺ T cells suppressed by CD14 monocytes.

### 7-2. Immune activation by anti-VISTA antibody and anti-PD-1 antibody (IFN-γ)

To confirm the immune activation ability of the antibody prepared in Example 2-3, a Staphylococcal enterotoxin B (SEB) activation assay was performed using human PBMCs. The human PBMCs separated in Example 7-1 were added at 2.0x10⁵ cells per well to a 96-well plate, and SEB (Abion) 100 ng/mL, anti-VISTA antibody 10 µg/mL, and anti-PD-1 antibody (Pembrolizumab) 10 µg/mL were added to the cell culture medium and incubated at 37°C for 3 days. After 3 days, 100 µL of the supernatant was used for subsequent analysis. The amount of IFN-γ secretion through immune activation was measured by ELISA (Human IFN-gamma DuoSet ELISA, R&D systems, Cat. No. DY285B).

As a result, as shown in Figure 12, it was confirmed that the antibody exhibited immune activation ability and maximized IFN-γ secretion when co-administered with the anti-PD-1 antibody.

### 7-3. Recovery of immune suppression by anti-VISTA antibody (IFN-γ, TNF-α, and IL-6)

To confirm the recovery ability of immune suppression by the antibody prepared in Example 2-3, a Staphylococcal enterotoxin B (SEB) activation assay was performed using VISTA protein and human PBMCs. Human VISTA protein (hVISTA-Fc, RND systems, Cat. No 7126-B7) was coated at a concentration of 5 µg/mL on a 96-well plate overnight at 4°C. The next day, the plate was washed three times with PBS, and the human PBMCs separated in Example 7-1 were added at 2.0x10⁵ cells per well to the 96-well plate. SEB (Abion) 100 ng/mL and anti-VISTA antibody diluted from 20 µg/mL at a 1/4 ratio were added to the cell culture medium and incubated at 37°C for 3 days. After 3 days, 100 µL of the supernatant was used for subsequent analysis. The amounts of IFN-γ, TNF-α, and IL-6 secretion through immune activation were measured using Human IFN-gamma DuoSet ELISA (R&D systems, Cat. No. DY285B), Human TNF-alpha DuoSet ELISA (R&D systems, Cat. No. DY210), and Human IL-6 DuoSet ELISA (R&D systems, Cat. No. DY206), respectively.

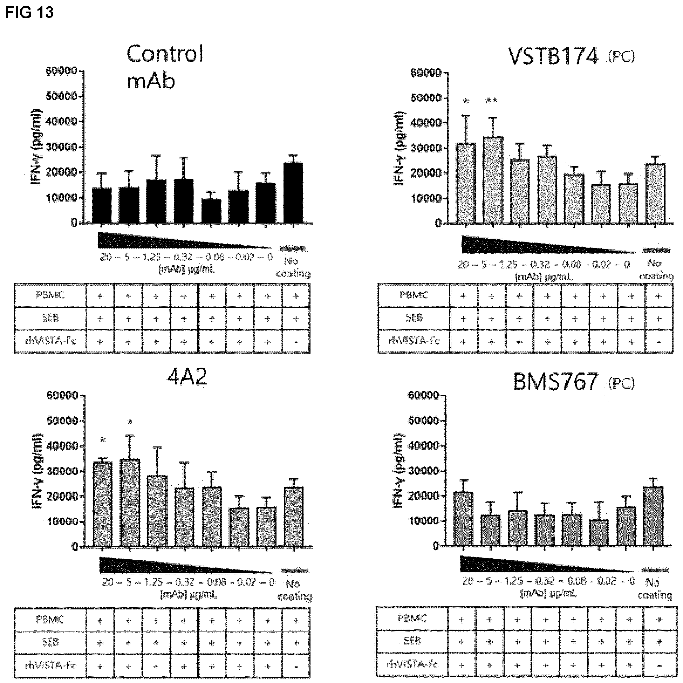

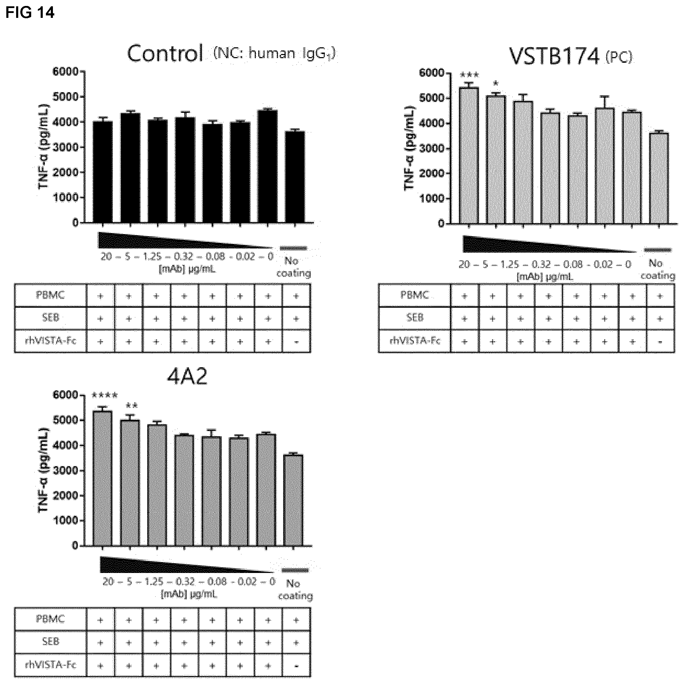

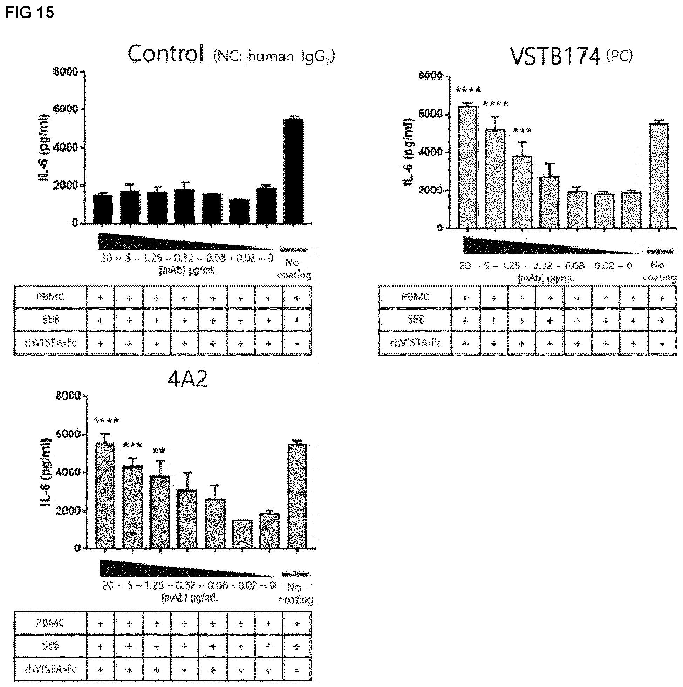

As a result, as shown in Figures 13 to 15, it was confirmed that the antibody restored the secretion of IFN-γ (Figure 13), TNF-α (Figure 14), and IL-6 (Figure 15) suppressed by VISTA protein in the concentration-dependent manner.

### 7-4. Confirmation of immune activation by anti-VISTA antibody through mixed lymphocyte reaction (MLR) test

To confirm the immune activation ability of the antibody prepared in Example 2-3, a mixed lymphocyte reaction (MLR) assay was conducted using PBMCs from different donors. The PBMCs to be used as stimulators were treated with mitomycin C to inhibit cell proliferation through human PBMCs isolated in Example 7-1. PBMCs from another donor to be used as responders and PBMCs to be used as stimulators were each added at 1.0x10⁵ cells per well in a 96-well plate, and the anti-VISTA antibody was treated to a concentration of 20 µg/ml (and diluted in a 1/4 ratio). The plate was incubated at 37°C for 5 days. After 5 days, 100 µl of the supernatant was collected for subsequent analysis. The amount of IL-6 secreted through immune activation was measured using ELISA (Human IL-6 DuoSet ELISA, R&D systems, Cat. No. DY206).

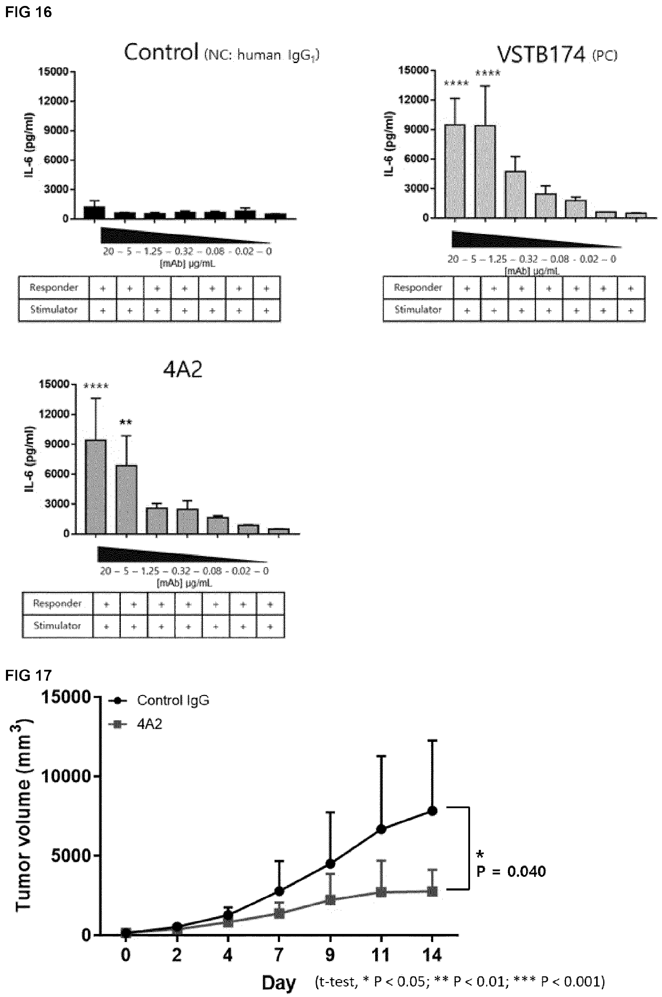

As a result, as shown in Figure 16, it was confirmed that the antibody increased IL-6 secretion in a concentration-dependent manner.

### Example 8. Confirmation of in vivo anti-cancer efficacy of the antibody

To confirm the *in vivo* anti-cancer efficacy of the anti-VISTA antibody, the prepared anti-VISTA antibody was injected into human VISTA protein-introduced mice induced with cancer, and changes in tumor size were observed.

First, 5x10⁵ MC38 cells, a mouse colon cancer cell line, were subcutaneously injected into human VISTA protein-introduced mice. When the average tumor size reached 140 mm³, the mice were randomly assigned to the control group and the antibody administration group, with 6 mice per group. The control group was administered IgG, while the antibody administration group was administered the 4A2 antibody at a dose of 3 mg/kg, three times a week for a total of 2 weeks. Tumor size was then measured. Tumor size was measured using calipers and calculated as long axis x (short axis)² mm³.

As a result, as shown in Figure 17, it was confirmed that the tumor size in the 4A2 antibody administration group was statistically significantly reduced compared to the control group (t-test, p value=0.04).

As described above, the present invention provides an antibody or a fragment thereof that binds to human VISTA protein. The antibody of the present invention binds to VISTA of T cells, thereby inhibiting the inactivation of T cells by cancer cells, allowing T cells to attack cancer cells, while also exerting a strong ADCC effect through NK cells. Therefore, it exhibits remarkable efficacy as an anticancer agent, making it highly industrially applicable.

## Claims

1. An antibody or a fragment thereof that binds to human VISTA protein selected from the group consisting of:
i) an antibody comprising a heavy chain variable region (V_{H}) comprising a complementarity determining region (CDR) H1 comprising the amino acid sequence represented by SEQ ID NO: 1, a complementarity determining region (CDR) H2 comprising the amino acid sequence represented by SEQ ID NO: 2, a complementarity determining region (CDR) H3 comprising the amino acid sequence represented by SEQ ID NO: 3, and a light chain variable region (V_{L}) comprising a complementarity determining region (CDR) L1 comprising the amino acid sequence represented by SEQ ID NO: 4, a complementarity determining region (CDR) L2 comprising the amino acid sequence represented by SEQ ID NO: 5, and a complementarity determining region (CDR) L3 comprising the amino acid sequence represented by SEQ ID NO: 6;
ii) an antibody comprising a heavy chain variable region (V_{H}) comprising a complementarity determining region (CDR) H1 comprising the amino acid sequence represented by SEQ ID NO: 9, a complementarity determining region (CDR) H2 comprising the amino acid sequence represented by SEQ ID NO: 10, a complementarity determining region (CDR) H3 comprising the amino acid sequence represented by SEQ ID NO: 11, and a light chain variable region (V_{L}) comprising a complementarity determining region (CDR) L1 comprising the amino acid sequence represented by SEQ ID NO: 12, a complementarity determining region (CDR) L2 comprising the amino acid sequence represented by SEQ ID NO: 13, and a complementarity determining region (CDR) L3 comprising the amino acid sequence represented by SEQ ID NO: 14;
iii) an antibody comprising a heavy chain variable region (V_{H}) comprising a complementarity determining region (CDR) H1 comprising the amino acid sequence represented by SEQ ID NO: 17, a complementarity determining region (CDR) H2 comprising the amino acid sequence represented by SEQ ID NO: 18, a complementarity determining region (CDR) H3 comprising the amino acid sequence represented by SEQ ID NO: 19, and a light chain variable region (V_{L}) comprising a complementarity determining region (CDR) L1 comprising the amino acid sequence represented by SEQ ID NO: 20, a complementarity determining region (CDR) L2 comprising the amino acid sequence represented by SEQ ID NO: 21, and a complementarity determining region (CDR) L3 comprising the amino acid sequence represented by SEQ ID NO: 22;
iv) an antibody comprising a heavy chain variable region (V_{H}) comprising a complementarity determining region (CDR) H1 comprising the amino acid sequence represented by SEQ ID NO: 25, a complementarity determining region (CDR) H2 comprising the amino acid sequence represented by SEQ ID NO: 26, a complementarity determining region (CDR) H3 comprising the amino acid sequence represented by SEQ ID NO: 27, and a light chain variable region (V_{L}) comprising a complementarity determining region (CDR) L1 comprising the amino acid sequence represented by SEQ ID NO: 28, a complementarity determining region (CDR) L2 comprising the amino acid sequence represented by SEQ ID NO: 29, and a complementarity determining region (CDR) L3 comprising the amino acid sequence represented by SEQ ID NO: 30;
v) an antibody comprising a heavy chain variable region (V_{H}) comprising a complementarity determining region (CDR) H1 comprising the amino acid sequence represented by SEQ ID NO: 33, a complementarity determining region (CDR) H2 comprising the amino acid sequence represented by SEQ ID NO: 34, a complementarity determining region (CDR) H3 comprising the amino acid sequence represented by SEQ ID NO: 35, and a light chain variable region (V_{L}) comprising a complementarity determining region (CDR) L1 comprising the amino acid sequence represented by SEQ ID NO: 36, a complementarity determining region (CDR) L2 comprising the amino acid sequence represented by SEQ ID NO: 37, and a complementarity determining region (CDR) L3 comprising the amino acid sequence represented by SEQ ID NO: 38; and
vi) an antibody comprising a heavy chain variable region (V_{H}) comprising a complementarity determining region (CDR) H1 comprising the amino acid sequence represented by SEQ ID NO: 41, a complementarity determining region (CDR) H2 comprising the amino acid sequence represented by SEQ ID NO: 42, a complementarity determining region (CDR) H3 comprising the amino acid sequence represented by SEQ ID NO: 43, and a light chain variable region (V_{L}) comprising a complementarity determining region (CDR) L1 comprising the amino acid sequence represented by SEQ ID NO: 44, a complementarity determining region (CDR) L2 comprising the amino acid sequence represented by SEQ ID NO: 45, and a complementarity determining region (CDR) L3 comprising the amino acid sequence represented by SEQ ID NO: 46.

2. The antibody or fragment thereof according to claim 1, wherein the antibody is selected from the group consisting of:
i) an antibody comprising a heavy chain variable region comprising the amino acid sequence from the 1st to 117th of SEQ ID NO: 7 and a light chain variable region comprising the amino acid sequence from the 1st to 108th of SEQ ID NO: 8;
ii) an antibody comprising a heavy chain variable region comprising the amino acid sequence from the 1st to 117th of SEQ ID NO: 15 and a light chain variable region comprising the amino acid sequence from the 1st to 108th of SEQ ID NO: 16;
iii) an antibody comprising a heavy chain variable region comprising the amino acid sequence from the 1st to 118th of SEQ ID NO: 23 and a light chain variable region comprising the amino acid sequence from the 1st to 107th of SEQ ID NO: 24;
iv) an antibody comprising a heavy chain variable region comprising the amino acid sequence from the 1st to 118th of SEQ ID NO: 31 and a light chain variable region comprising the amino acid sequence from the 1st to 107th of SEQ ID NO: 32;
v) an antibody comprising a heavy chain variable region comprising the amino acid sequence from the 1st to 115th of SEQ ID NO: 39 and a light chain variable region comprising the amino acid sequence from the 1st to 106th of SEQ ID NO: 40; and
vi) an antibody comprising a heavy chain variable region comprising the amino acid sequence from the 1st to 119th of SEQ ID NO: 47 and a light chain variable region comprising the amino acid sequence from the 1st to 106th of SEQ ID NO: 48.

3. The antibody or fragment thereof according to claim 1, wherein the antibody is selected from the group consisting of:
i) an antibody comprising a heavy chain consisting of SEQ ID NO: 7 and a light chain consisting of SEQ ID NO: 8;
ii) an antibody comprising a heavy chain consisting of SEQ ID NO: 15 and a light chain consisting of SEQ ID NO: 16;
iii) an antibody comprising a heavy chain consisting of SEQ ID NO: 23 and a light chain consisting of SEQ ID NO: 24;
iv) an antibody comprising a heavy chain consisting of SEQ ID NO: 31 and a light chain consisting of SEQ ID NO: 32;
v) an antibody comprising a heavy chain consisting of SEQ ID NO: 39 and a light chain consisting of SEQ ID NO: 40; and
vi) an antibody comprising a heavy chain consisting of SEQ ID NO: 47 and a light chain consisting of SEQ ID NO: 48.

4. The antibody or fragment thereof according to claim 1, wherein the fragment is selected from the group consisting of diabody, Fab, Fab', F(ab)₂, F(ab')₂, Fv and scFv.

5. A polynucleotide encoding the antibody or fragment thereof according to claim 1.

6. A recombinant vector comprising the polynucleotide according to claim 5.

7. A transformed cell line comprising the vector according to claim 6.

8. A method for producing an antibody or a fragment thereof that binds to human VISTA protein, comprising culturing the cell line according to claim 7 under conditions in which the polynucleotide is expressed to produce a polypeptide comprising a light chain and heavy chain variable region; and recovering the polypeptide from the cell line or culture medium in which the cell line was cultured.

9. A pharmaceutical composition for preventing or treating cancer comprising the antibody or fragment thereof according to claim 1 as an active ingredient.

10. The pharmaceutical composition according to claim 9, wherein the cancer is selected from the group consisting of breast cancer, colorectal cancer, head and neck cancer, colon cancer, skin cancer, pancreatic cancer, lung cancer, gastric cancer, ovarian cancer, prostate cancer, bladder cancer, urethral cancer, liver cancer, kidney cancer, clear cell sarcoma, melanoma, brain and spinal cord tumor, brain cancer, thymoma, mesothelioma, esophageal cancer, bile duct cancer, testicular cancer, germ cell tumor, thyroid cancer, parathyroid cancer, cervical cancer, endometrial cancer, lymphoma, myelodysplastic syndromes (MDS), myelofibrosis, acute leukemia, chronic leukemia, multiple myeloma, Hodgkin's disease, endocrine cancer, and sarcoma.

11. Use of the antibody or fragment thereof according to claim 1 for manufacturing a pharmaceutical composition for treating cancer.

12. A method for treating cancer comprising administering an effective amount of a pharmaceutical composition comprising the antibody or fragment thereof according to claim 1 as an active ingredient to a subject in need thereof.
